# EUROPEAN PATENT APPLICATION

(11) **EP 3 184 101 A1**
(43) Date of publication of application: **28.06.2017**
(21) Application number: 15201645.7
(22) Date of filing: 21.12.2015
(51) Int. Cl.: A61K 9/16, A61K 9/48, A61K 45/06, A61K 31/195, A61K 31/198, A61K 31/194

(54) **LEVODOPA AND CARBIDOPA MODIFIED RELEASE COMPOSITION**

(71) Applicant: SANDOZ AG, 4056 Basel (CH)
(72) Inventor: The designation of the inventor has not yet been filed
(74) Representative: Kluschanzoff, Harald

(57) **Abstract**

The present invention belongs to the field of pharmaceutical industry and refers to a multilayered coated particle that comprises levodopa, carbidopa in the form of carbidopa monohydrate and carboxylic acid (tartaric acid). The present invention further refers to a pharmaceutical composition and a dosage form comprising one or more of said multilayered coated particle(s). Moreover, the present invention refers to a process for preparing said particle, pharmaceutical composition and dosage form, and to the use of said dosage form in the treatment of Parkinson's disease.

## Description

### Field of the invention

The present invention belongs to the field of pharmaceutical industry and relates to a multilayered coated particle comprising carboxylic acid, levodopa, and carbidopa. The present invention further refers to a pharmaceutical composition and a dosage form comprising one or more of said multilayered coated particle(s). Moreover, the present invention refers to a process for preparing said particle, pharmaceutical composition and dosage form, and to the use of said dosage form in the treatment of Parkinson's disease (PD).

### Description of the background art

As it is commonly known in the art of pharmaceutics, it is not only a relevant factor contributing to a possible success of treating certain diseases which pharmaceutically active ingredient (API) is chosen for the treatment of said disease, but also the treatment regimen and in particular the release profile of the administered API(s) after administration can be a decisive factor. Special attention has to be paid when a tight release/dissolution profile of the API(s) and possibly further ingredients such as pharmaceutically acceptable excipients has to be obtained.

An example that is known in the art of such API whose treatment regimen has to be tightly controlled are combinations of the APIs levodopa (LD) and a decarboxylase inhibitor, typically carbidopa (CD). LD and CD are considered as the standard APIs for treating degenerative disorders of the central nervous system (CNS), particularly for treating Parkinson's disease (PD). Administration of LD is challenging, as it undergoes rapid decarboxylation by tissues other than the brain, which is the actual desired target location and site of action of LD. Hence, in order to prevent decarboxylation of LD, it is known in the art to additionally administer a decarboxylase inhibitor, usually CD. This decarboxylase inhibitor inhibits the decarboxylation of peripheral LD and it does not cross the blood brain barrier. Further, its decarboxylating activity is limited to the extracerebral tissue. Due to these beneficial properties, it has become common art to administer LD in combination with CD, in order to make sure that sufficient LD is available for transport into the brain.

It is further known that patients suffering from degenerative diseases of the CNS, and in particular patients suffering from PD, may have periods where motility is impaired, followed by periods characterized by immobility.

In order to be able to control such periods of PD, and/or in order to essentially avoid the occurrence of unwanted periods of certain diseases in general, it may be advantageous to provide pharmaceutical compositions that exhibit a specific API release profile.

With this regard, US 8,377,474 B2 discloses controlled release multiparticulate controlled release solid oral formulation comprising levodopa and carbidopa, a decarboxylase inhibitor and a carboxylic acid, and further comprising controlled release components and immediate release components.

WO 2014/006571 A2 discloses extended release pharmaceutical dosage forms of carbidopa and levodopa, comprising a first component comprising a first portion of CD and LD; a second component comprising a second portion of said APIs; and a third component comprising a third portion of CD, LD and carboxylic acid.

Despite the above described dosage forms, there is still a need and thus it is an object of the present invention to provide for improved pharmaceutical compositions such as layered particles, or oral dosage forms comprising said layered particles, as well as improved methods for producing the same.

### Summary of the invention

The present invention provides the following aspects, subject-matters and preferred embodiments, which respectively taken alone or in combination, contribute to solving the object of the present invention:
(1) A multilayered coated particle comprising, preferably consisting of, from the inside to the outside, in the order from i) to vi):
   i) a core comprising, preferably consisting of, one or more carboxylic acids;
   ii) a controlled (extended) release coating layer comprising one or more rate-controlling polymers which is/are practically insoluble at a pH in the range of from 1 to 8, and optionally one or more coating additives, preferably directly on said core;
   iii) one or more Modified Release System (MRS) layer combination(s), preferably directly positioned on said controlled release coating layer (ii), wherein each MRS layer comprises
      (iiia) an API coating layer,
      comprising levodopa and carbidopa (preferably carbidopa monohydrate) and one or more immediate release or modified release film forming polymers and/or one or more coating additives, or
      an API coating layer, which is a combination of two API coating layers,
      one comprising levodopa and one comprising carbidopa, and each comprising one or more immediate release or modified release film forming polymers and/or one or more coating additives;
      and
      (iiib) a controlled (extended) release coating layer,
      comprising one or more rate-controlling polymers which is/are practically insoluble at a pH in the range of from 1 to 8 and optionally one or more coating additives;
      wherein, from the inside to the outside, coating layer (iiia) precedes coating layer (iiib);
      (iiic) optionally further a coating layer combination having two layers (α) and (β), wherein layer (α) comprises one or more carboxylic acid(s), one or more film forming polymers and optionally anti-tacking agent and plasticizer, and layer (β) is a separating seal coat comprising one or more immediate release or modified release film forming polymers and optionally one or more coating additives, preferably anti-tacking agents and/or plasticizer,
      wherein said layer α of the optional coating layer combination (iiic) can be present either prior or after the API coating layer (iiia),
      with the proviso that in case optional layer combination (iiic) is provided, the separating seal coat β is positioned between layer α and the API coating layer (iiia);
      wherein the MRS layer combination(s) (iii) can be present up to 5 times in a consecutive manner;
   iv) optionally, a further API coating layer as defined in (iiia), preferably directly positioned on the MRS layer combination(s) (iii), if present;
   v) a controlled (preferably delayed) release coating layer (v) comprising one or more rate controlling polymer(s) with pH dependent solubility or a mixture of rate controlling polymer(s) with pH dependent solubility and rate controlling polymer(s) which is/are practically insoluble at a pH in the range of from 1 to 8, and one or more coating additives, preferably directly positioned on the optional further API coating layer (iv), or on the Modified Release System (MRS) layer combination(s) (iii), if API coating layer (iv) is absent; and
   vi) an immediate release API coating layer comprising levodopa, carbidopa monohydrate, one or more immediate release film forming polymers and/or one or more coating additives, preferably not covered by a further layer.
   The carboxylic acid is neither levodopa nor a decarboxylase inhibitor such as carbidopa.
   In a preferred embodiment, i) to vi) directly follow each other, i.e. there is no additional layer present at any position between i) to vi).
(2) The multilayered coated particle according to (1), wherein the carboxylic acid is tartaric acid, and/or wherein the coating layers (ii), (iiib), and (v) additionally comprise talc, and/or wherein the polymers and coating additives, present in the same layer, are different from each other.
   In particular, the polymers and coating additives being present in coating layers (ii), (iiia), (iiib), (iiic), (iv), (v), and (vi) are different from each other.
(3) The multilayered coated particle according to (1) or (2), wherein
   (I) at least one, preferably all, of said rate-controlling polymer(s) which is/are practically insoluble at a pH in the range of from 1 to 8 is/are selected from the group consisting of cellulose derivatives such as ethylcellulose; polymethacrylate esters/co-polymers, such as meth- /acrylate copolymers with trimethyl-ammonioethyl-methacrylate as functional group (e.g. polymers from the Eudragit® RL series, such as Eudragit® RL 30 D, PO, 100, 12,5, and polymers from the Eudragit® RS series, such as Eudragit® RS 30 D, PO, 100, 12,5), and such as neutral polymers of meth- /acrylates (e.g. polymers from the Eudragit® NE series, such as Eudragit® NE 30 D, and 40 D, and polymers from the Eudragit® NM series, such as Eudragit® NM 30 D); waxes; lipids; polyvinyl chloride; polyethylene; vinyl acetate/vinyl chloride copolymers; and polyamides;
   (II) the rate-controlling polymer(s) with pH-dependent solubility is/are pharmaceutically acceptable and have a pH dependent solubility within the pH range of from 1 to 8; preferably, at least one, preferably all, of said rate-controlling polymer(s) is/are selected from the group consisting of cellulose acetate phthalate (CAP); cellulose acetate trimellitate (CAT); carboxymethyl ethylcellulose (CMEC); hydroxypropylmethylcellulose phthalate (HPMCP); methacrylic acid and methyl methycrylate copolymers; fatty acids; waxes; shellack; and polyvinyl acetate phthalate (PVAP);
   (III) the coating additive(s) is/are pharmaceutically acceptable and at least one, preferably all, is/are selected from the group consisting of plasticizers such as triethylcitrate (TEC); appearance modifiers such as pigments; anti-tacking agents; permeability-modifying agents, e.g. soluble polymers such as polyvinylpyrrolidone (PVP), low viscosity (preferably less than 1000 mPa s of 2% aqueous solution as determined according to ASTM standards D1347 or D2363) HPMC (e.g. Pharmacoat 606), low viscosity (preferably less than 100 mPa s of 2% aqueous solution as determined according to ASTM standards D1347 or D2363) hydroxypropylcellulose (HPC), high viscosity (preferably at least 10000 mPa s of 2% aqueous solution as determined according to ASTM standards D1347 or D2363) HPMC, high viscosity (preferably 1000-4000 mPa s of 2% aqueous solution as determined according to ASTM standards D1347 or D2363) HPC, or polysaccharides;
   (IV) the film forming polymer(s) is/are pharmaceutically acceptable and is/are able to form a film and bind solids in dispersions into a coating, preferably the film forming polymer is an immediate release film forming polymer that has essentially no delaying or sustaining influence on API release, such as low viscosity HPMC (e.g. Pharmacoat 606), low viscosity HPC, or PVP;
      or the film forming polymer(s) is/are a modified release film forming polymer that is able to alter the API release, such as polymers that are practically insoluble in water, such as cellulose derivatives (e.g. ethylcellulose, preferably ethylcellulose having a very high content of added ethoxyl groups expressed as degree of substitution of 2.2-2.8, or 2.4-2.8), polymethacrylates, copolymers of methacrylic acid; polymers that form a matrix or gel, such as polysaccharides; or hydrophilic polymers that swell in contact with water and thereby form a hydrophilic matrix or a gel, such as high viscosity HPMC (e.g. Methocel K4M - K100M), high viscosity HPC (e.g. Klucel MXF).
(4) The multilayered coated particle according to any of (1) to (3), wherein
   (A) the controlled (extended) release coating layer (ii) comprises ethylcellulose, preferably ethylcellulose N10, as a rate-controlling polymer and low viscosity HPMC, preferably HPMC Pharmacoat 606, as a coating additive;
   (B) the coating layer (iiia) comprises low viscosity HPMC, preferably HPMC Pharmacoat 606, as film forming polymer;
   (C) the controlled (extended) release coating layer (iiib) comprises ethylcellulose, preferably ethylcellulose N10, as a rate-controlling polymer and low viscosity HPMC, preferably HPMC Pharmacoat 606, as coating additive;
   (D) the coating layer (iiic), if present, comprises low viscosity HPMC, preferably HPMC Pharmacoat 606, or PVP as a film-forming polymer in the layer comprising carboxylic acid(s), and anti-tacking agent and/or plasticizer in the seal coat;
   (E) the controlled (preferably delayed) coating layer (v) comprises two different types of methacrylic acid copolymers as rate controlling polymers, preferably methacrylic acid copolymer Type B (e.g. Eudragit® S 100) and methacrylic acid copolymer Type A (e.g. Eudragit® L 100), and TEC and talc as coating additive; and/or
   (F) the immediate release coating layer (vi) comprises HPMC Pharmacoat 606 as film forming polymer, and/or one or more disintegrants as coating additive(s), such as crospovidone, croscarmellose sodium, or sodium starch glycolate.
(5) The multilayered coated particle according to (4), wherein the weight ratio of low viscosity HPMC, preferably HPMC Pharmacoat 606, to ethylcellulose, preferably ethylcellulose N10, (e) in coating layer (ii) is in a range of from 1:0.5 to 1:15, preferably 1:1 to 1:9, more preferably in a range of from 1:2 to 1:6, even more preferably in a range of from 1:3 to 1:5, and most preferably in a range of from 1:4 to 1:5; (ee) in coating layer (iiib) is in a range of from 1:0.5 to 1:15, preferably 1:1 to 1:9, more preferably in a range of from 1:1 to 1:3, even more preferably in a range of from 1:1 to 1:1.5.
(6) The multilayered coated particle according to any of (1) to (5), wherein the weight ratio of the two different types of methacrylic acid copolymers as rate controlling polymers, preferably Eudragit S 100 and Eudragit L 100, is in a range of from 1:0.05 to 0.05:1, preferably 1:0.25 to 1:2.5, more preferably is in a range of from 1:0.25 to 1:2.0, even more preferably in a range of from 1:0.5 to 1:2.0, further preferred in a range of from 1:0.5 to 1:1.5, and most preferably in a range of from 1:0.5 to 1:1.
(7) The multilayered coated particle according to any of (1) to (6), wherein the MRS coating layer combination(s) comprise(s) carbidopa monohydrate and levodopa in an amount of equal to or more than 60 wt.-% and equal to or less than 90 wt.-%, preferably in an amount of equal to or more than 70 wt.-% and equal to or less than 80 wt.-%, and more preferably in an amount of equal to or more than 75 wt.-% and equal to or less than 80 wt.-%, based on the total amount of carbidopa monohydrate and levodopa being present in the multilayered coated particle.
(8) The multilayered coated particle according to any of (1) to (7), wherein the carbidopa monohydrate and the levodopa respectively have a maximum mean particle size of d(0.9) of about 35µm, preferably the carbidopa monohydrate and the levodopa respectively have a mean particle size of d(0.9) in the range of from 1 µm to 30 µm, as determined by laser diffraction technology on the basis of the volumetric mean value.
(9) The multilayered coated particle according to any of (1) to (8), comprising two MRS coating layer combinations.
(10) Pharmaceutical composition comprising the multilayered coated particles according to any of (1) to (9), and at least one further pharmaceutically acceptable excipient selected from the group consisting of filler, binder, lubricant, glidant, and disintegrant.
(11) An oral dosage form, preferably a tablet, sachet or a capsule, more preferably a capsule, comprising the pharmaceutical composition according to (10) or one or more of the multilayered coated particles according to (1) to (9).
(12) Process for the preparation of a multilayered coated particle as defined in any of (1) to (9), comprising the steps of
   a) providing the core (i);
   b) applying the controlled release coating layer (ii) by using a dispersion comprising one or more rate-controlling polymer(s) and optionally one or more coating additive(s), preferably directly on said pellet core;
   c) applying the API coating layer (iiia) by using an organic or aqueous dispersion or a dispersion containing organic solvent(s) and water, preferably an organic dispersion having a water content of less than 20 v/v, comprising levodopa and carbidopa monohydrate, and one or more immediate release or modified release film forming polymer(s) and/or one or more coating additive(s), preferably directly on the controlled release coating layer (ii);
   d) applying the controlled, preferably delayed or extended, release coating layer (iiib) by using a coating dispersion comprising one or more rate-controlling polymer(s) which is/are practically insoluble at a pH in the range of from 1 to 8, and optionally one or more coating additive(s), preferably directly on the API coating layer (iiia);
   e) optionally, applying layer (α) of the coating layer combination (iiic) by using a dispersion of carboxylic acid and one or more film forming polymer(s), preferably immediate release film forming polymer(s), and optionally anti-tacking agent and plasticizer; and
      applying layer (β) of the coating layer combination (iiic) directly prior or after the API coating layer (iiia) by using a coating dispersion comprising one or more immediate release or modified release film forming polymer(s) and/or one or more coating additive(s);
      wherein steps c) to e) can be repeated up to 4 times;
   f) optionally, applying the API coating layer (iv) by using a dispersion comprising levodopa and carbidopa monohydrate, and one or more immediate release or modified release film forming polymer(s) and/or one or more coating additive(s), preferably directly on the MRS layer combination(s) (iii);
   g) applying the controlled, preferably delayed, release coating layer (v) by using a dispersion comprising one or more rate controlling polymer(s) with pH dependent solubility or a mixture of rate controlling polymers with pH dependent solubility and rate controlling polymer(s) which is/are practically insoluble at a pH in the range of from 1 to 8, and one or more coating additive(s), preferably directly on the API coating layer (iv), or on the Modified Release System (MRS) layer combination(s) (iii), if API coating layer (iv) is absent; and
   h) applying the immediate release API coating layer (vi) by using a dispersion comprising carbidopa monohydrate, levodopa, one or more immediate release film forming polymer(s), and/or one or more coating additive(s), preferably directly on the controlled, preferably delayed, release coating layer (v).
      Optionally, after each coating step measures are taken to remove agglomerates that may have formed during coating process. An example of such a measure is screening.
      In a preferred embodiment, the polymer(s), coating additive(s), anti-tacking agent, and plasticizer is as disclosed elsewhere herein, preferably as disclosed in the preceding items.
(13) The process of (12), wherein the carboxylic acid is tartaric acid.
(14) The process of (12) and (13), wherein the core either comprises, or consists of, tartaric acid crystals, and/or is prepared by layering or extrusion spheronisation.
(15) The process according to any of (12) to (14), wherein the respective layers are applied by spray coating technique.
(16) The process according to any of (12) to (15), wherein, if the dispersion of step c) is an aqueous dispersion, this aqueous dispersion has a pH of below 3.
(17) The process according to any of (12) to (16), wherein the carbidopa monohydrate and the levodopa respectively have a maximum mean particle size of d0.9 of about 35µm, preferably the carbidopa monohydrate and the levodopa respectively have a mean particle size of d0.9 in the range of from 1 µm to 30 µm, as determined by laser diffraction technology on the basis of the volumetric mean value.
(18) The process according to any of (12) to (17) for preparing a multilayered coated particle according to (4), wherein the dispersion used for preparing the controlled release coating layer (ii) has a solid content in a range of from 3 to 15 w/w% and an ethyl cellulose to HPMC ratio in a range of from 1:1 to 10:1 and/or wherein the ratio of the weight of the controlled release coating layer (ii) to the weight of the pellet core (i) plus the weight of the controlled release coating layer (ii) is in a range of from 5% to 40%, preferably 10% to 30%, more preferably is in a range of from 15 to 25%, further preferred is about 20%.
(19) The process according to any of (12) to (18), wherein the dispersion used for preparing the/one of the MRS layer combination(s) (iii) has a solid content in a range of from 3 to 15 w/w% and an ethyl cellulose to HPMC ratio in a range of from 2:1 to 10:1 and/or wherein the ratio of the weight of the MRS layer combination(s) (iii) to the weight of the pellet core (i) plus the weight of the controlled release coating layer (ii) plus the weight of the MRS layer combination(s) (iii) is in a range of from 5% to 40%, preferably 5% to 20%, more preferably is in a range of from 7 to 15%, further preferred is about 10%.
(20) The process according to any of (12) to (19), wherein the dispersion used for preparing the/one of the MRS layer combination(s) (iii) has a solid content in a range of from 3 to 15 w/w% and an ethyl cellulose to HPMC ratio in a range of from 1:1 to less than 2:1 and/or wherein the ratio of the weight of the MRS layer combination(s) (iii) to the weight of the pellet core (i) plus the weight of the controlled release coating layer (ii) plus the weight of the MRS layer combination(s) (iii) is in a range of from 5% to 20%, preferably is in a range of from 7 to 15%, further preferred is about 10%.
(21) The multilayered coated particle according to any of (1) to (9), the pharmaceutical composition according to (10), or the oral dosage form of (11) for use in a method of treating Parkinson's disease.

### Definitions

Within the meaning of the present invention, the term "API" denotes "pharmaceutically active ingredient". The APIs that are referred to in the present invention are levodopa ("LD") and carbidopa (CD). Carboxylic acid, for example tartaric acid, is not an API. Rather, it is an acidifying agent. Carbidopa is a decarboxylase inhibitor that can prevent the breakdown of levodopa before it can reach the brain and take effect. Thus, usually, levodopa is administered in combination with carbidopa. In the present invention, carbidopa is administered in the form of carbidopa monohydrate.

Unless specified otherwise, within the meaning of the present invention, the term "soluble" has to be understood as "water soluble".

Unless specified otherwise, the term "insoluble" has to be understood as "practically insoluble in water" and defines that the solubility of the API in water is less than or equal to 0.1 mg/ml. Preferably, the solubility of such an API in water is less than or equal to 0.001 mg/ml.

The term "pharmaceutically acceptable excipient" as used herein includes excipients that are conventionally used in pharmaceutical compositions. The pharmaceutically acceptable excipients can for instance be polymers or lipids, and can for instance be surfactants, solubility enhancers, fillers, lubricants, binders, disintegrants, coloring agents, plasticizers, pore formers, and film forming polymers.

In order to provide API release at defined, specific desired target sites, and/or to provide for a certain desired release/dissolution profile of the API(s), specific pharmaceutically acceptable excipients have to be chosen. Said specifically chosen excipients provide for a targeted release of the API at the desired target site, and/or a certain desired release/dissolution profile.

The release/dissolution profile of an API can be adjusted by choosing defined pharmaceutically acceptable excipients. By choosing specific excipients, desired modified release profiles can be generated. Usually, the specific excipients are rate controlling pharmaceutically acceptable excipients with pH dependent solubility, or excipients which are practically insoluble at a pH in the range of from 1 to 8. The respective excipients belonging to each group are known to a person skilled in the art. It is also possible to mix the respective excipients in order to arrive at a certain release profile.

The term "dissolution rate" or "dissolution profile" of an API refers to the amount of drug substance (API) that goes into solution per unit time under standardized conditions of liquid/solid interface, temperature and solvent composition.

The term "release profile" of an API refers to the amount of drug substance (API) that is set free (released) from the dosage form in the course of time under standardized conditions.

There are different release profiles that can be obtained by applying specific pharmaceutically acceptable excipients:
In the following, the different release profiles are defined in connection with API. This is not to be understood as limiting. Rather, the below definitions broadly apply to the respectively assessed release profile of any component that is of interest, if appropriate.

The expression "immediate release" as used herein defines that the API (in particular carbidopa and levodopa) is released substantially immediately after oral administration, with substantially complete dissolution within one hour.

The term "modified release" pharmaceutical formulation (e.g. dosage form) as used herein defines that the rate and/or site of release of the API is different from that of the immediate release pharmaceutical formulation administered by the same route. Examples of modified release pharmaceutical formulations are delayed release, extended release, and multiphasic release pharmaceutical formulation.

The term "delayed release" defines that the API is released at a time other than promptly after administration. In other words, the release of the API from the pharmaceutical formulation (dosage form, coating layer) is delayed for a certain period after administration or application of the pharmaceutical formulation. The subsequent release is similar to that of an immediate release pharmaceutical formulation.

The term "controlled release" describes various types of modified release pharmaceutical formulations (dosage forms, coating layers), including delayed release (e.g. immediate release at a specific pH corresponding to a lag time of dissolution after ingestion until the gastrointestinal region is reached where the specific pH occurs), sustained release, prolonged release and extended release.

The term "extended release" (similar to "sustained release" and "prolonged release") defines that the API(s) is made available over a prolonged period of time following ingestion - compared to an immediate release -, in particular, an extended release dosage form dissolves *in vivo* or *in vitro* for a period of more than one hour. *In vitro* dissolution can be tested by using Apparatus 2 (paddle) as described in USP chapter 711 (Official December 1, 2011) using water at pH 7 as medium. An extended API release allows a reduction in dosing frequency and reduction of fluctuations in plasma concentrations as compared to when the drug is present in an immediate-release pharmaceutical formulation (dosage form). API release at a predetermined rate in order to maintain a constant reduced API concentration (versus a spike-profile obtained with immediate release) for a specific period of time with minimum side effects.

The term "multiphasic release" defines that the release of the API(s) occurs in multiple phases. There is a (first) phase of API release that is determined by the immediate release API fraction providing a therapeutic API level shortly after API administration. The (second) extended release phase provides the API fraction required to maintain an effective therapeutic level for a prolonged period. The phases of immediate release and extended release can be present multiple times. If there is one immediate release phase and one extended release phase, the API release is biphasic.

A pharmaceutically acceptable excipient (such as polymer or lipid) that is "practically insoluble at a pH in the range of from 1-8" is an excipient that has a very low solubility in gastrointestinal fluid over the entire pH range of from about 1 to about 8, as determined in the test as indicated below. This pH range is the range that can be found in the GI tract of humans. Solubility can be determined by using the shake flask method in media with different pH values or simply by providing saturated aqueous solutions of excipient in water at pH 1, 3, 5 and 8, evaporating the water and weighing the amount of excipient. More specifically, the term "practically insoluble" denotes that greater than or equal to 10,000 parts of water are required to dissolve 1 part of solute.

The extended release of the API occurs for instance through the permeability of the membrane/coating (diffusion process) and is essentially time-dependent and pH independent. The respective excipients that can be used in order to provide the desired API release are rate-controlling, this means that they provide for the release the API and/or carboxylic acid(s) in a controlled manner. This practically pH independent, controlled release can be obtained by the use of defined pharmaceutically acceptable excipients and methods that are known to a skilled person, such as by means of applying insoluble matrix systems, soluble matrix systems, membrane-controlled delivery/release, or osmosis controlled release.

In general, in insoluble matrix systems a controlled release of the API is obtained by the API diffusing out of said matrix system, or through the permeable membrane, respectively. According to one embodiment, the excipient(s) is/are chosen such that a matrix system is formed, out of which the API diffuses. Examples of such pharmaceutically acceptable excipients are known to the skilled person and are for instance matrix-forming lipids such as waxes, with pore forming agents (channeling agents), and non-soluble polymers such as ethyl cellulose or polyvinyl acetate. Further examples are listed elsewhere herein.

In soluble (or hydrophilic) matrix systems, diffusion from or erosion of the matrix controls the release of the API. The principle is that upon contact with water, the matrix swells, and/or the matrix material dissolves. The API is then exposed to the external fluids such as gastrointestinal fluid, and is mixed with or dissolved into said fluids. Examples of such pharmaceutically acceptable excipients that are able to form soluble (hydrophilic) matrixes are known to the skilled person and are for instance eroding waxes or hydrophilic polymers, or alginates and high viscosity hydroxypropylmethylcellulose (HPMC).

In membrane controlled delivery, the API diffuses through the membrane. Usually, a film coating using a permeable membrane is used for this purpose, or a film coating with a combination of a nonsoluble film and soluble pore formers such as sugars or low viscosity HPMC.

A pharmaceutically acceptable excipient that has "pH dependent solubility" is any pharmaceutically acceptable excipient whose solubility differs over the pH range of from 1 to 8. As described above, solubility can be determined by using the shake flask method in media with different pH values or simply by providing saturated aqueous solutions of excipient in water at pH 1, 3, 5 and 8, evaporating the water and weighing the amount of excipient. More specifically, the term " pH dependent solubility " means that 2 or more solubility values at 1, 3, 5 and 8 differ by more than 50%. The respective excipients that can be used in order to provide the desired API release are rate-controlling, this means that they release the API in a controlled manner. By using respective suitable excipients, a targeted release of the API can be obtained at desired pH values, which, in turn, provides for a targeted release at desired sites exhibiting the respective pH value in the gastrointestinal (GI) tract and its gastrointestinal fluid. The gastrointestinal fluid is fluid being present in the gastrointestinal tract (GI). The GI comprises the duodenum having a pH of equal to or greater than about 5.5, the ileum having a pH of equal to or greater than about pH 7.0, the jejunum having a pH of about 6-7, and the stomach having a pH of between about 1 to 5. Hence, depending on the respective pH dependent solubility of the pharmaceutically acceptable excipients used, the release profile can be adjusted and a targeted release at desired sites of the GI tract can be obtained.

Examples of rate-controlling excipients with pH-dependent solubility are listed elsewhere herein.

Within the meaning of the present invention, the expression "film forming polymer" is any pharmaceutically acceptable excipient that is able to form a film and that binds solids (such as API) in dispersion into a coating. There exist immediate release film forming polymers and modified release film forming polymers.

Within the meaning of the present invention, the expression "immediate release film forming polymers" denotes polymers that have no substantial delaying, sustaining influence on API release. Suitable polymers are known to the skilled person and are further listed elsewhere herein.

Within the meaning of the present invention, the term "modified release film forming polymer" denotes polymers that are able to alter the API release. Suitable polymers are known to a skilled person and are further listed elsewhere herein.

Within the meaning of the present invention, the term "Modified Release System" ("MRS") denotes a combination of coating layers: From the inside to the outside, the MRS comprises an API coating layer and a controlled release coating layer. Optionally, a further coating layer combination with two layers (layer (α) and layer (β)) can be present: One layer comprises carboxylic acid(s), one or more film forming polymers and optionally anti-tacking agent and plasticizer (layer (α)), and the other coating layer is a seal layer (seal coat; layer (β)). The layer comprising the carboxylic acid(s) of the further coating layer combination, if present, can be present prior or after the API comprising layer of the MRS. It is then, however, obligatory that between the API coating layer of the MRS and the layer comprising the carboxylic acid(s) an additional seal layer coating is present. The components of the seal coating layer do not have a substantial, preferably no measurable, effect on the release of the API(s). It is the purpose of the seal layer coating that the carboxylic acid layer and the API layer are physically separated in order to avoid detrimental effects of the carboxylic acid on the API(s) (such as degradation or the like).

Within the meaning of the present invention, the term "coating additives" denotes any pharmaceutically acceptable excipient that can be added in or on a coating in order to:
- Improve plasticity of the coating (e.g. plasticizer);
- Modify the appearance of the coating (e.g. pigments);
- Reduce the sticking and improve processability (e.g. anti tacking agents; pigments);
- Modify film permeability (e.g. soluble polymers such as PVP, HPMC, HPC; soluble organic (e.g. lactose, mannitol, and the like) or inorganic (e.g. salts) components).

It is known to a skilled person that one and the same pharmaceutically acceptable excipient can have more than one function, such as permeability-modifying agents can also be film forming polymers. A person skilled in the art will be able to assess which function(s) the respective excipient exhibits.

The term "about" as used herein refers to any value which lies within the range defined by a variation of up to 10% of the value.

The term "carboxylic acid" as used herein refers to all organic acids and salts thereof. Suitable examples of organic acids can be selected from the group consisting of tartaric acid, adipic acid, succinic acid, aitric acid, benzoic acid, maleic acid, acetic acid, ascorbic acid, edetic acid, fumaric acid, lactic acid, malic acid, oleic acid, sorbic acid, stearic acid, palmitic acid, glutaric acid, glutamic acid, mandelic acid, or mixtures thereof. Preferably, the carboxylic acid is tartaric acid. The salts include, but are not limited to, alkaline and earth alkaline earth metal salts, such as sodium, potassium, lithium, calcium, strontium, barium, and antimony; the ammonium salts; or the alkanolamine salts.

The carboxylic acid of the present invention can be present in a pellet core (or pellet) or can be layered on a pellet core (or pellet). Further, it can additionally be present in a coating layer. The latter, i.e. the presence of the carboxylic acid in a coating layer, is optional.

The terms "pellet core", "core", or "pellet(s)" within the meaning of the present invention defines essentially spherical cores. This geometric shape ensures that essentially uniform coating is obtained. As carboxylic acid pellet cores, carboxylic acid crystals (e.g. tartaric acid crystals) can be used, or pellets of carboxylic acid (e.g. tartaric acid) prepared by layering or extrusion spheronisation process. The mean pellet core size can be 50 - 1500 µm, preferably 200 - 1000 µm, more preferably 300 - 700 um. Measurement of pellet core size can be carried out as described below.

In the present invention, for example the (maximum) mean particle size of carbidopa monohydrate and levodopa is defined. In general, the particle size distribution of a powder, or granular material, or particles, is a list of values or a mathematical function that defines the relative amounts of particles present, sorted according to size. The nomenclature describing the particle size distribution is herein referred to as "d(0.9)". For example, a d(0.9) of about 35µm means that 90 % (by volume) of the particles have a size less than or equal to 35µm, as tested by any conventional method such as laser diffraction method (e.g. laser diffraction Malvern). When measuring the particle size distribution by laser diffraction Malvern, an appropriate amount of sample is put into a gently mixing chamber filled with water. From this chamber, the sample continuously flows through a measuring chamber and the particle size distribution is determined by laser diffraction. A suitable method is described in European Pharmacopeia, Chapter 2.9.31 Particle size analysis by laser light diffraction.

Within the meaning of the present invention, the term "pharmaceutical composition" defines a combination of multilayered coated particle(s) of the present invention, and one or more further pharmaceutically acceptable excipient(s).

The pharmaceutical composition can thus denote a dosage form, e.g. a final dosage form, such as a tablet, capsule, or sachet, or any intermediate that arises (is generated) during the manufacturing process of the dosage form.

Within the meaning of the present invention, the term "dosage form" refers to means by which drug molecules are delivered to sites of action within the body. Examples of sites of action are for instance the gastrointestinal tract GI). An oral dosage form is a dosage form that can be administered orally. Examples of oral dosage forms are tablets, capsules, dragees, or sachets.

It can also be that there is (are) no further pharmaceutically acceptable excipient(s) added in addition to the multilayered coated particles. In this case, said particles are for instance simply encapsulated into a hard or soft shell, preferably into a hard shell. The resulting final dosage form would then be a capsule.

Within the meaning of the present invention, the term "capsule" denotes a solid dosage form comprising the multilayered coated particle according to the present invention, wherein this dosage form has hard or soft shells. A capsule can be of various shapes, and it is intended for oral administration. Thus, a capsule is an example of a solid oral dosage form. The shell is usually formed from gelatin or HPMC. It is also possible that in addition to the multilayered coated particle according to the present invention, the capsule comprises additional, further pharmaceutically acceptable excipients. These further excipients are not present in the multilayered coated particles, but outside, and still within the (final) dosage form.

Within the meaning of the present invention, the term "sachet" denotes single-dose presentations of powder that are intended to be administered to the patient as such, to be taken in or with water.

The term "further pharmaceutically acceptable excipient(s)" refers to pharmaceutically acceptable excipient(s) that is (are) present outside the multilayered coated particles, but within the (final) dosage form. In other words, this further pharmaceutically acceptable excipient(s) is (are) present in the phase outside of the multilayered coated particles, i.e. in the extraparticulate phase.

The amount of API/carboxylic acid/carbidopa monohydrate that is to be set free can be adjusted by introducing respectively desired amounts of API/ carboxylic acid/carbidopa monohydrate in the respective layers.

Consequently, by applying the present invention, a single, individual multilayered coated particle is provided that exhibits a tailored release profile of LD and CD, and of the carboxylic acid.

### Detailed description of the invention

The present invention is now described in more detail by preferred embodiments and examples, which are however presented for illustrative purpose only and shall not be understood as limiting the scope of the present invention in any way.

The present invention provides a multilayered coated particle, comprising multiple layers which, in turn, respectively represent controlled release layer(s), API containing layer(s), immediate release layer(s), and optionally carboxylic acid containing layer(s) and, if carboxylic acid containing layer(s) is (are) present, seal layer(s) that are located between the API layer and the carboxylic acid layer.

The multilayered coated particle according to the present invention already comprises all release stages of multicomponent systems known in prior art that comprise the APIs levodopa (LD) and carbidopa (CD) and carboxylic acid such as tartaric acid. Consequently, and in other words, one single multilayered coated particle of the present invention exhibits essentially the same release profile as the entirety of the multilayered coated particles that are present in the final dosage form. Further, the multilayered coated particle according to the present invention is able to provide for a specific, desired release profile and/or dissolution profile which allows for the adequate treatment of patients suffering from general diseases of the CNS, preferably the treatment of PD. In particular, the multilayered particles according to the present invention can provide for a modified release of LD and CD, thereby providing steady state of LD plasma concentration over a prolonged period of time, with immediate onset of action. This desired release profile can be arrived at by one and the same type of coated particle in a final dosage form, without the need for preparing multiple types of components which each type exhibiting a different release profile of the API(s) and/or the carboxylic acid.

In prior art, in order to arrive at an adequate, suitable release profile of the respective APIs and the carboxylic acid, it was necessary to combine multiple, different types of separated components such as granules, pellets or particles in one pharmaceutical composition or dosage form, respectively, with each type of component providing for a different function such as a different release time of API(s) and/or carboxylic acid. This was necessary in order to ensure an immediate onset of action, accompanied by steady state plasma levels of LD over a prolonged period of time.

Contrary thereto, the multilayered pellets of the present invention already comprise in one individual pellet the different types of layers that are necessary in order to provide the full range of the desired release profile of API(s) and carboxylic acid, such as an immediate onset of action and a steady state plasma level of LD and CD over a prolonged period of time. In other words, the multilayered particles of the present invention already comprise, in each individual pellet, all desired release stages of CD, LD, and carboxylic acid that are necessary for suitable, proper treatment of diseases of the CNS (such as PD), i.e. all release stages of corresponding multicomponent systems that are known in prior art.

By applying this approach, difficult and time-consuming preparation of final dosage forms, such as capsules, can be avoided. This is because all individual, single multilayered pellets already comprise all release stages of API and carboxylic acid. Therefore, contrary to prior art techniques that are currently used for preparing final dosage forms and that require elaborate measures for providing the proper amount of each type of component in the final dosage form, the multilayered pellets of the present invention do not require such sophisticated techniques. This is because each multilayered coated pellet that is present in the pharmaceutical composition or final dosage form, respectively, essentially corresponds to each other. Thus, it can for instance be avoided to define the ratio of pellet components in the pharmaceutical composition/final formulation, which requires the use of specialized machines, with sophisticated and expensive weight controlling systems which should ensure appropriate filling of each component. It is a further difficulty and disadvantage that commercially available multicomponent filling machines (e.g. capsule filling machines) usually have a limited ability of regulation of single component filling.

A further option in the prior art to fill the different types of components is to mix all components in the appropriate ratio prior to combining them into the final dosage form. However, this approach is highly prone to segregation of the respective components and thus inaccurate dosing of each individual component. The cumulative release profile of such combined systems strongly depends e.g. on the ratio of pellet components in the final formulation; providing the respectively correct ratio of each component is however difficult. Again, by applying the present invention, advantageously difficult and/or time consuming prior art approaches can be avoided as each individual particle essentially corresponds to each other, in respect to its ingredients and its release profile.

Additionally, by applying the present invention, content and/or component variations related to inaccurate multicomponent incorporation or segregation of a mixture of combined components, thus assuring uniformity of dosage forms in terms of LD and CD content and release profile of the (final) dosage forms.

Moreover, the production of the coated multilayered particle according to the present invention is improved as its manufacture involves only one type of process, i.e. fluid bed coating. Thus, it can be that only one piece of equipment is required for applying the coatings of the multilayered particle. This is contrary to prior art processes, where each component can be considered as unique product or type of component, respectively, and therefore requiring different production processes, analysis and release prior to the final incorporation (encapsulation) step.

Summed up, the multilayered coated particles of the present invention provide for an improved process for preparing pharmaceutical oral dosage forms.

Hence, the present invention represents an alternative dosage form comprising LD, CD and carboxylic acid, which enables modified (controlled) release of LD and CD and which provides steady state of LD plasma concentration over prolonged period of time with immediate onset of action. This is achieved in the following way:
- Initial LD and CD dose is released from outer immediate release layer (referred to herein as coating layer (vi)) which enables rapid onset LD plasma concentration immediately after dosing;
- delayed release coating with pH dependent solubility (referred to herein as coating layer (v)) enables pH controlled release of subsequent LD CD portions, it prevents release of subsequent LD and CD portions in the stomach;
- an optional second API layer (referred to herein as coating layer (iv)) is released immediately after passage of multiparticulates from stomach into duodenum when pH increases above pH dependent polymer solubility value;
- MRS layer combination(s) (also referred to herein as "MRS layer(s)") (1 layer, at most 5 layer combinations) (referred to herein as coating layer (iii), comprising coating layer (iiia), (iiib), and optionally (iiic)) enable pH independent, step-wise controlled (extended) release of subsequent portions of LD and CD. MRS layer(s) prolong absorption of levodopa in lower part of the intestine, thereby maintaining effective levodopa plasma levels for longer period of time. It means that MRS layer(s) effectively provide constant supply of LD and CD maintenance dose;
- practically pH independent controlled release layer (referred to herein as coating layer (ii)) which controls release of TA and simultaneously acts as a separate layer effectively preventing direct contact between incompatible TA and CD resulting in improved stability of the formulation.

Due to the multi-layered approach desired release profile of LD and CD and TA is characteristic for individual pellet what is of great benefit for encapsulation of the final formulation as the pellets can be encapsulated using a regular one component capsule filling machine.'

The present invention thus relates to a multilayered coated particle comprising, from the inside to the outside, in the order from i) to vi), the following core (i) and the coating layers (ii) to (vi) as disclosed in the following:
As starting particle, a core (i) comprising, preferably consisting of, one or more carboxylic acids, is present. The core (i) preferably comprises tartaric acid as carboxylic acid, and
further preferred, tartaric acid is the only carboxylic acid being present in said core.

Directly after this core (i), a controlled release coating layer (ii) is present, which comprises one or more rate-controlling polymer(s) which is/are practically insoluble at a pH in the range of from 1 to 8. This pH independent controlled release layer controls the release of the carboxylic acid(s) and simultaneously acts as a separate layer effectively preventing direct contact between the incompatible carboxylic acid(s) and the API layer, therefore resulting in an improved stability of the pharmaceutical formulation.

The controlled release coating layer (ii) is followed, preferably directly, by one or more MRS layer combination(s) (iii) comprising an API coating layer (iiia), a controlled, preferably delayed or extended, release coating layer (iiib), wherein, form the inside to the outside, coating layer (iiia) precedes coating layer (iiib), and optionally a further coating layer combination (iiic).

The API coating layer (iiia) comprises levodopa and carbidopa monohydrate, and one or more immediate release or modified release film forming polymers and/or one or more coating additives.

The controlled, preferably delayed or extended, release coating layer (iiib) comprises one or more rate-controlling polymer(s) which is/are practically insoluble at a pH in the range of from 1 to 8, and optionally one or more coating additives, wherein, form the inside to the outside, coating layer (iiia) precedes coating layer (iiib).

The optional further coating layer (iiic) has two layers, wherein one layer comprises one or more carboxylic acid(s), one or more film forming polymers and optionally anti-tacking agent and plasticizer, and one layer is a separating seal coat comprising one or more immediate release or modified release film forming polymers and optionally one or more coating additives, preferably anti-tacking agents and/or plasticizer.

The one layer comprising one or more carboxylic acid(s) of the optional coating layer (iiic) can be present either prior or after the API coating layer (iiia), with the proviso that in case optional layer (iiic) is present, the separating seal coat is positioned between the layer comprising the one or more carboxylic acid(s) and the one or more film forming polymer(s), and the API coating layer (iiia).

The MRS layer can be present up to 5 times in order to achieve the desired multi-(bi)-phasic release of levodopa. Preferably, the MRS layer is present two times, which means that the multilayered coated particle comprises two MRS coating layer combinations. The respective portions of the API in each layer are adjusted in order to achieve the desired drug release. This (multi-) MRS layered approach enables simple but very accurate adjustment of respective API portions at different release stages. Further, this approach enables simple adjustment of drug release by modifying the respective portion of API in each system, by modifying the composition of extended release and delayed release coatings (amount of coating; polymer ratios in the coatings; coating additives).

Optional, the MRS layer combination(s) is followed, preferably directly, by a further API coating layer (iv). This API coating layer (iv) is preferably as defined in (iiia).

After coating layer (iii) or, if present, after coating layer (iv), a controlled, preferably delayed, release coating layer (v) comprising one or more rate controlling polymers with pH dependent solubility or a mixture of polymers with pH dependent solubility and polymer(s) which is/are practically insoluble at a pH in the range of from 1 to 8, and one or more coating additives, is present.

Finally, an immediate release API coating layer (vi) comprising levodopa, carbidopa monohydrate, one or more immediate release film forming polymers and/or one or more coating additives, is present. In a preferred embodiment, this layer (vi) is not covered by a further layer. It is further preferred that the immediate release layer (vi) does not comprise carboxylic acid such as tartaric acid.

In the following, the polymers/coating additives that can be present in the coating layers (ii) to (vi) are described in more detail:
By using polymers which are practically insoluble at a pH in the range of from 1 to 8, it is ensured that the release of the carboxylic acid(s) being present in the core is essentially time-dependent (and not pH dependent).

The controlled release coating layer (ii) preferably comprises rate-controlling polymer(s) which is/are practically insoluble at a pH in the range of from 1 to 8 and is/are pharmaceutically acceptable. Preferably, at least one, preferably all, of the rate-controlling polymer(s) being present in the multilayered coated particle is/are selected from the group consisting of cellulose derivatives such as ethylcellulose; polymethacrylate esters/copolymers, such as meth- /acrylate copolymers with trimethyl-ammonioethyl-methacrylate as functional group (e.g. polymers from the Eudragit® RL series, such as Eudragit® RL 30 D, PO, 100, 12,5, and polymers from the Eudragit® RS series, such as Eudragit® RS 30 D, PO, 100, 12,5), and such as neutral polymers of meth- /acrylates (e.g. polymers from the Eudragit® NE series, such as Eudragit® NE 30 D, and 40 D, and polymers from the Eudragit® NM series, such as Eudragit® NM 30 D; waxes; lipids; polyvinyl chloride; polyethylene; vinyl acetate/vinyl chloride copolymers; and polyamides.

The rate-controlling polymer(s) with pH-dependent solubility is/are pharmaceutically acceptable and have a pH dependent solubility within the pH range of from about 1 to about 8; preferably, at least one, preferably all, of said rate-controlling polymer(s) is/are selected from the group consisting of cellulose acetate phthalate (CAP); cellulose acetate trimellitate (CAT); carboxymethyl ethylcellulose (CMEC); hydroxypropylmethylcellulose phthalate (HPMCP); methacrylic acid and methyl methycrylate copolymers; fatty acids; waxes; shellack; and polyvinyl acetate phthalate (PVAP).

The coating additive(s) is/are pharmaceutically acceptable and at least one, preferably all, is/are selected from the group consisting of plasticizers such as triethylcitrate (TEC); appearance modifiers such as pigments; anti-tacking agents; permeability-modifying agents, e.g. soluble polymers such as polyvinylpyrrolidone (PVP), low viscosity HPMC (e.g. Pharmacoat 606), or low viscosity hydroxypropylcellulose (HPC), or hydrophilic polymers such as high viscosity HPMC or high viscosity HPC, or polysaccharides.

The film forming polymer(s) is/are pharmaceutically acceptable and is/are able to form a film and bind solids in dispersions into a coating. In a preferred embodiment, the film forming polymer is an immediate release film forming polymer that has essentially no delaying or sustaining influence on API release, such as low viscosity HPMC (e.g. Pharmacoat 606), low viscosity HPC, or PVP.

The film forming polymer(s) can also be a modified release film forming polymer that is able to alter the API release, such as polymers that are practically insoluble in water, such as cellulose derivatives (e.g. ethylcellulose), polymethacrylates, copolymers of methacrylic acid; polymers that form a matrix or gel, such as polysaccharides; or hydrophilic polymers that swell in contact with water and thereby form a hydrophilic matrix or a gel, such as high viscosity HPMC or high viscosity HPC.

In a preferred embodiment, the multilayered coated particle comprises one or all the following coating layers in addition to core (i), with said coating layers comprising the following components:
The controlled (extended) release coating layer (ii) comprises ethylcellulose (EC), preferably ethylcellulose N10, as a rate-controlling polymer and low viscosity HPMC, preferably HPMC Pharmacoat 606, as a coating additive;
the coating layer (iiia) comprises low viscosity HPMC, preferably HPMC Pharmacoat 606, as film forming polymer;
the coating layer (iiib) comprises ethylcellulose, preferably ethylcellulose N10, as a rate-controlling polymer and low viscosity HPMC, preferably HPMC Pharmacoat 606, as coating additive;
the coating layer (iiic), if present, comprises low viscosity HPMC, preferably HPMC Pharmacoat 606, or PVP as a film-forming polymer in the layer comprising carboxylic acid(s), and anti-tacking agent and/or plasticizer in the seal coat;
the coating layer (v) comprises two different types of methacrylic acid copolymers as rate controlling polymers, preferably methacrylic acid copolymer Type B (e.g. Eudragit® S 100) and methacrylic acid copolymer Type A (e.g. Eudragit® L 100), and TEC as coating additive; and/or
the coating layer (vi) comprises HPMC Pharmacoat 606 as film forming polymer, and one or more disintegrants as coating additive(s), such as crospovidone, croscarmellose sodium, or sodium starch glycolate.

Additionally, the coating layer(s) (ii), (iiib) and/or (v) comprise(s) talc.

In a further embodiment, the polymers and coating additives that are present in the same coating layer are different from each other.

It is additionally preferred that the weight ratio of low viscosity HPMC, preferably HPMC Pharmacoat 606, to ethylcellulose, preferably ethylcellulose N10, in coating layer (ii) is in a range of from 1:1 to 1:9, preferably in a range of from 1:2 to 1:6, more preferably in a range of from 1:3 to 1:5, and even more preferably in a range of from 1:4 to 1:5; and/or in coating layer (iiib) is in a range of from 1:1 to 1:9, preferably in a range of from 1:1 to 1:3, more preferably in a range of from 1:1 to 1:1.5.

In a further preferred embodiment, the weight ratio of the two different types of methacrylic acid copolymers as rate controlling polymers, preferably Eudragit S 100 and Eudragit L 100, is in a range of from 1:0.1 to 1:6, preferably 1:0.25 to 1:2.5, further preferred in a range of from 1:0.25 to 1:2.0, more preferably in a range of from 1:0.5 to 1:2.0, even more preferably in a range of from 1:0.5 to 1:1.5, and most preferably in a range of from 1:0.5 to 1:1.

In general, the MRS coating layer combination(s) comprise(s) the APIs carbidopa monohydrate and levodopa in any desired amount. Preferably, carbidopa monohydrate and levodopa are comprised in an amount of equal to or more than 60 wt.-% and equal to or less than 90 wt.-%, preferably in an amount of equal to or more than 70 wt.-% and equal to or less than 80 wt.-%, and more preferably in an amount of equal to or more than 75 wt.-% and equal to or less than 80 wt.-%, based on the total amount of carbidopa monohydrate and levodopa being present in the multilayered coated particle.

The respective amount of the API, as well as the respective amount (ratio) of the excipients, can be determined by any suitable mean that is known to a skilled person. For instance, PAT (process analytical technologies)- techniques such as NIR (near-infrared) spectroscopy can be used to monitor and control the amount of levodopa and carbidopa monohydrate, therefore assuring accurate dosing. The same technique can also be used for monitoring the respective amount of the pharmaceutically acceptable excipient(s) (such as rate controlling polymer(s)). By doing so, the desired release profile and also the desired target site can be controlled and ensured.

It is additionally preferred that the carbidopa monohydrate and the levodopa respectively have a maximum mean particle size of d(0.9) of about 35µm, preferably the carbidopa monohydrate and the levodopa respectively have a mean particle size of d(0.9) in the range of from 1 µm to 30 µm, as determined by laser diffraction technology on the basis of the volumetric mean value. By using this mean particle size, high process efficiency (in terms of comparably high yield and comparably high drug loading) can be achieved.

The present invention also refers to a pharmaceutical composition comprising the multilayered coated particles according to the present invention, and at least one further pharmaceutically acceptable excipient. This pharmaceutically acceptable excipient can be any suitable excipient that is known to a skilled person. Usually, said one or more further excipient(s) is (are) chosen inter alia with regard to the final dosage form the pharmaceutical composition can be, or is, formulated into. For instance, if the final dosage form is a tablet, then the excipients are chosen such that they do not interact in an undesired manner with the multilayered coated particle(s) of the present invention. "Undesired manner" means that no negative interaction such as degradation of the multilayered coated particle(s) takes place. Furthermore, breaking of the layers of the coated particles during compression should be avoided, e.g. by choosing proper excipients. Such further excipients can for instance selected from the group consisting of filler, binder, lubricant, glidant, and disintegrant.

The same is true if the final dosage form is a capsule: Any further pharmaceutically acceptable excipient(s), if present at all, are chosen such that there is no negative interaction with regard to the capsule shell. Of course, also the pharmaceutically acceptable excipients that are present in the multilayered coated particle(s) should be chosen such that they do not cause deterioration of the shell of the capsule, if they should come into contact with the shell.

The present invention further refers to an oral dosage form, preferably a solid oral dosage form, that comprises the pharmaceutical composition of the present invention, or one or more of the multilayered coated particles of the present invention. In a preferred embodiment, the oral dosage form is a tablet, sachet, or capsule. Preferably, the oral dosage form is solid, and is a capsule.

The formulation of the multilayered coated particle(s) or of the pharmaceutical composition of the present invention into oral dosage forms, preferably solid oral dosage forms, can be carried out by any suitable means that is known to a person skilled in the art.

For instance, a tablet can be formulated by applying wet or dry granulation, which comprises the steps of first forming granules and then tableting the granules, or by applying direct compression.

In general, capsules can be formulated by filling the respective ingredients of the capsule (e.g. particles, such as the multilayered coated particles of the present invention) into a capsule filling machine.

By applying the multilayered coated particles of the present invention, it has been surprisingly found out that the encapsulating process can be improved. This is because the inventive individual particle already exhibits the desired release profile, i.e. all release stages of the prior art multicomponent system, into an individual particle. Thus, highly complex multicomponent encapsulation can be avoided since the particles of the present invention can be encapsulated by a single component encapsulation process using regular volumetric based capsule filling machine. By doing so, also content and/or component variation due to inaccurate multicomponent encapsulation or segregation of a mixture of combined component can be avoided, thereby assuring satisfying content uniformity of the dosage form in respect of LD and CD content and release profile of the capsule.

Contrary thereto, the prior art approach to achieve steady release of LC and CD with rapid onset of action in combination with carboxylic acid is to combine several types of pellet components with distinct release profile into final dosage forms. The cumulative release profile of such prior art combined systems strongly depends on the ratio of pellet components in the final formulation (dosage form) which requires the use of specialized capsule filling machines, with sophisticated weight controlling systems which ensures appropriate filling of each component. By applying the present invention, such time consuming and complex filling procedures can be avoided, as well as problems associated therewith.

Sachets can be formulated by any suitable method that is known to a skilled person, e.g. by filling the sachets based on the volumetric principle using appropriate equipment. Formulating the pharmaceutical composition into capsules is preferred.

The present invention further refers to a process for the preparation of a multilayered coated particle according to the present invention.

By applying this process, the manufacture of dosage forms comprising the multilayered particles of the present invention can be improved. This is because said multilayered coated particles are substantially the same, and consequently, the particles can be prepared by applying the same process, namely fluid bed coating, which requires only one piece of equipment. This is contrary to prior art methods, where a dosage form represents a multicomponent product comprising various types of components, with each type exhibiting a different composition. Thus, each type of component can be considered as a unique product requiring an individual production process, and quality controls prior to formulating them into the final dosage form, such as prior to the final encapsulation process.

In a first step a) of the process for the preparation of the multilayered coated particle of the present invention, a core (i) is provided. This core comprises, or consists of, carboxylic acid such as tartaric acid. The core can be prepared by applying layering techniques or extrusion spheronisation. Tartaric acid pellets are commercially available. Also crystals of tartaric acid can be used. These techniques are known to a skilled person. In short, in the extrusion spheronization technique, the core is prepared by mixing the carboxylic acid and pharmaceutical excipient enabling extrusion spheronisation (e.g. MCC) with a granulating liquid, followed by extruding the resulting mass through extruder followed by spheronizer.

In the solution/suspension/powder layering technique, the core is prepared e.g. by coating the carboxylic acid solution/dispersion/powder on suitable spheres (starting cores - such as sugar spheres) in a fluidized bed processor.

In a next step b), the extended release coating layer (ii) is applied by using a dispersion comprising one or more rate-controlling polymers and optionally one or more coating additives. In a preferred embodiment, coating layer (ii) is applied directly, i.e. without one or more coatings/layers in between (i) and (ii), on the pellet core (i).

The controlled release coating layer (ii) modifies the release of the carboxylic acid, e.g. tartaric acid, and contemporary acts as physical barrier between the carboxylic acid and the API coating layer.

In a next step c), the API coating layer (iiia) is applied by using an organic or aqueous dispersion comprising levodopa and carbidopa monohydrate, and one or more immediate release or modified release film forming polymers and/or one or more coating additives. LC and CD are unstable in water. Thus, organic solvent or acidic aqueous media with a pH of below 3 for the coating dispersion is preferred. It is further preferred that coating layer (iiia) is applied directly on the controlled release layer (ii), i.e. without one or more coatings/layers in between (ii) and (iiia). In order to obtain high yield and/or high drug (API) loading, preferably API with small particle size (mean particle size of d0.9 of about 35µm) is used. The mean particle size of carbidopa monohydrate preferably is also in this range.

In a next step d), the controlled, preferably extended, release coating layer (iiib) is applied by using a coating dispersion comprising one or more rate-controlling polymer(s) which is/are practically insoluble at a pH in the range of from 1 to 8, and optionally one or more coating additives, preferably directly in the API coating layer (iiia).

In a next step e), which is optional, the first layer of the coating layer combination (iiic) is applied onto the coating layer (iiib), by using a dispersion of carboxylic acid and one or more film forming polymers, preferably immediate release film forming polymers, and optionally anti-tacking agent and plasticizer. The second layer of the coating layer combination (iiic) is applied directly prior or after the API coating layer (iiia) by using a coating dispersion comprising one or more immediate release or modified release film forming polymers and/or one or more coating additives.

The coating layer systems that results from applying steps c) to e) represent the MRS layer combination. Steps c) to e) can be repeated up to 4 times, so as to arrive at a maximum of 5 MRS layer combinations.

In a next step f), which is optional, the API coating layer (iv) is applied, by using a dispersion comprising levodopa and carbidopa monohydrate, and one or more immediate release or modified release film forming polymers and/or one or more coating additives, preferably directly on the MRS layer combination(s) (iii).

In a next step g), the controlled, preferably delayed, release coating layer (v) is applied onto on the API coating layer (iv), or on the MRS layer combination(s) (iii), if API coating layer (iv) is absent. The coating layer (v) is applied by using a dispersion comprising one or more rate controlling polymers with pH dependent solubility or a mixture of polymer(s) with pH dependent solubility and polymer(s) which is/are practically insoluble at a pH in the range of from 1 to 8, and one or more coating additives. Preferably, the controlled release coating layer (v) is applied directly on the API coating layer (iv) or on the MRS layer combination(s) (iii), if API coating layer (iv) is absent.

In step h), the immediate release API coating layer (vi) is applied by using a dispersion comprising carbidopa monohydrate, levodopa, one or more immediate release film forming polymers, and/or one or more coating additives. In a preferred embodiment, the API coating layer (vi) is applied directly on the controlled release coating layer (v).

In a preferred embodiment, after step(s) a), b), c), d), e), f), g), and/or h), measures can be taken to remove agglomerates that may have formed during the coating process. An example of such a measure is carrying out a screening of the respectively obtained pellets.

Further preferred, the carboxylic acid used in the process is tartaric acid.

With regard to the polymers and coating additives that are used in the process according to the present invention, in particular with regard to the preferred ones, reference is made to the description elsewhere herein.

It is further preferred that the respective coating layers are applied by suitable spray coating techniques that are known to a person skilled in the art. Preferably, the spray coating process is carried out in fluid bed dried using bottom spray approach.

It is further preferred that the carbidopa monohydrate and the levodopa respectively have a maximum mean particle size of d0.9 of about 35µm, preferably the carbidopa monohydrate and the levodopa respectively have a mean particle size of d0.9 in the range of from 1 µm to 30 µm, as determined by laser diffraction technology on the basis of the volumetric mean value. This mean particle size contributes to high process efficiency, e.g. with regard to high yield and high drug loading.

It is further preferred that the controlled (extended) release coating layer (ii) of step b) comprises ethylcellulose, preferably ethylcellulose N10, as a rate-controlling polymer and low viscosity HPMC, preferably HPMC Pharmacoat 606, as a coating additive;
that the coating layer (iiia) of step c) comprises low viscosity HPMC, preferably HPMC Pharmacoat 606, as film forming polymer;
that the coating layer (iiib) of step d) comprises ethylcellulose, preferably ethylcellulose N10, as a rate-controlling polymer and low viscosity HPMC, preferably HPMC Pharmacoat 606, as coating additive;
that the coating layer (iiic) of step e), if present, comprises low viscosity HPMC, preferably HPMC Pharmacoat 606, or PVP as a film-forming polymer in the layer comprising carboxylic acid(s), and anti-tacking agent and/or plasticizer in the seal coat;
that the coating layer (v) of step g) comprises two different types of methacrylic acid copolymers as rate controlling polymers, preferably methacrylic acid copolymer Type B (e.g. Eudragit® S 100) and methacrylic acid copolymer Type A (e.g. Eudragit® L 100), and TEC as coating additive; and/or
that the coating layer (vi) of step h) comprises HPMC Pharmacoat 606 as film forming polymer, and one or more disintegrants as coating additive(s), such as crospovidone, croscarmellose sodium, or sodium starch glycolate.

Preferably, the dispersions used for preparing the controlled release coating layer (ii) has a solid content in a range of from 3 to 15 w/w% and an ethyl cellulose to HPMC ratio in a range of from 1:1 to 10:1, and/or a ratio of the weight of the controlled release coating layer (ii) to the weight of the pellet core (i) plus the weight of the controlled release coating layer (ii) is in a range of from 5 to 50% or 5 to 40%, preferably 10% to 30%, further preferred in a range of from 15 to 25%, further preferred about 20%.

Additionally preferred, the dispersion used for preparing the/one of the MRS layer combination(s) (iii) has a solid content in a range of from 3 to 15 w/w% and an ethyl cellulose to HPMC ratio in a range of from 2:1 to 10:1 and/or has a ratio of the weight of the MRS layer combination(s) (iii) to the weight of the pellet core (i) plus the weight of the controlled release coating layer (ii) plus the weight of the MRS layer combination(s) (iii) in a range of from 5 to 30%, preferably 5% to 20%, further preferred in a range of from 7 to 15%, even further preferred about 10%.

Additional preferred, the dispersion used for preparing the/one of the MRS layer combination(s) (iii) has a solid content in a range of from 3 to 15 w/w% and an ethyl cellulose to HPMC ratio in a range of from 1:1 to less than 2:1 and/or a ratio of the weight of the MRS layer combination(s) (iii) to the weight of the pellet core (i) plus the weight of the controlled release coating layer (ii) plus the weight of the MRS layer combination(s) (iii) in a range of from from 5 to 30%, preferably 5% to 20%, further preferred in a range of from 7 to 15%, even further preferred about 10%.

Finally, the present invention refers to an oral dosage form for use in a method of treating Parkinson's disease.

### Description of the figures

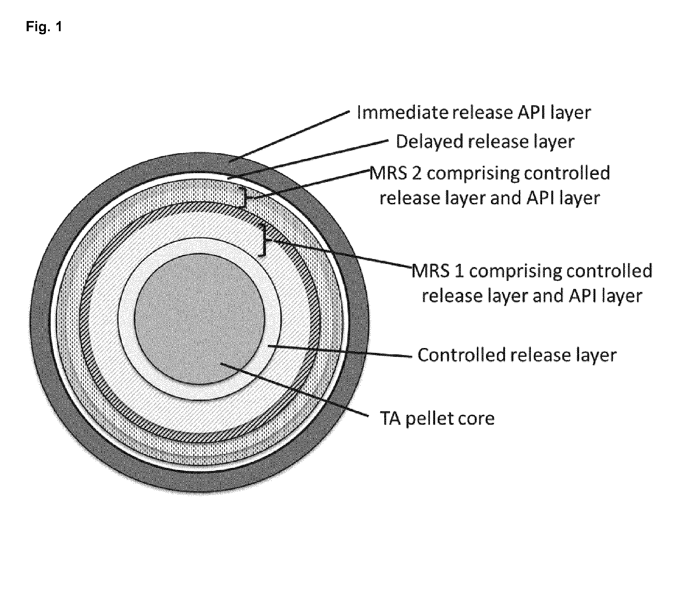

Fig. 1 represents a schematic presentation of a multilayered multiparticulate (multilayered coated particle) of LD, CD and TA according to the present invention.
   The depicted multilayered particle comprises a TA pellet core; a controlled (extended) release layer; two MRS (Modified Release System) layers respectively containing API layer and controlled (extended) release layer; a delayed release layer, and an immediate release layer.

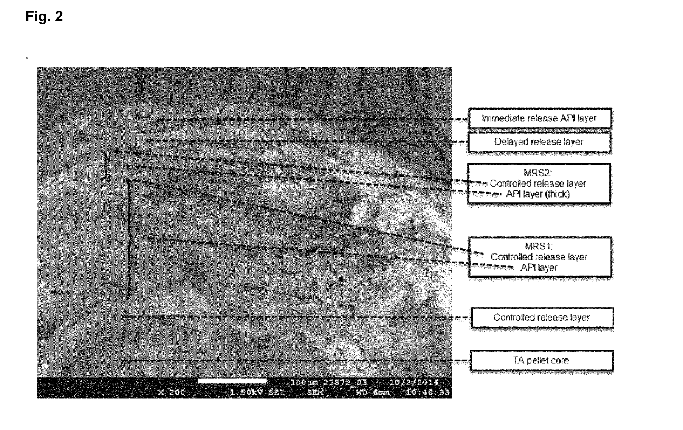

Fig. 2 represents an SEM image of multilayered multiparticulate (multilayered coated particle) of LD, CD and TA.
Fig. 3 shows the percent dissolved (mean values) of levodopa.
Fig. 4 shows percent dissolved (mean values) of carbidopa.
Fig. 5 shows percent dissolved (mean values) of tartaric acid.

### Methods

### Dissolution method

Dissolution method is designed to mimic gastrointestinal environment where the dosage form first reaches the stomach where acidic environment is present, and then, after one to two hours, the dosage form passes to duodenum and upper intestinal tract, where pH conditions are around pH 7.

First stage is 0.1 M HCI (pH 1) where gastric resistance is challenged and immediate release part of formulation is dissolved. At second stage Phosphate Buffer, pH 6.8 is applied.

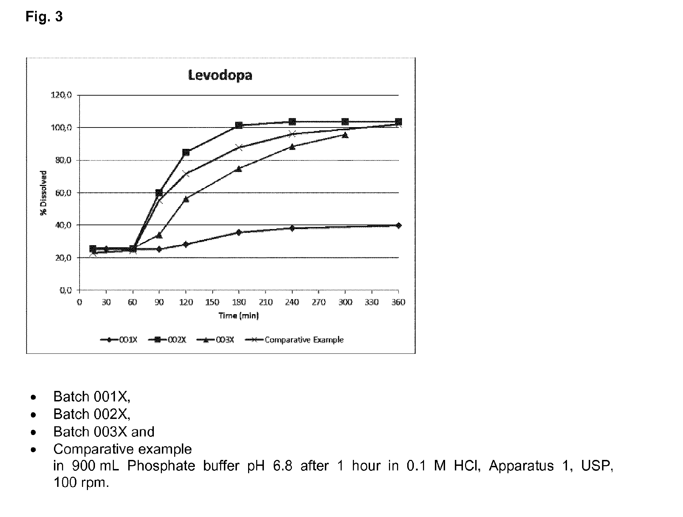

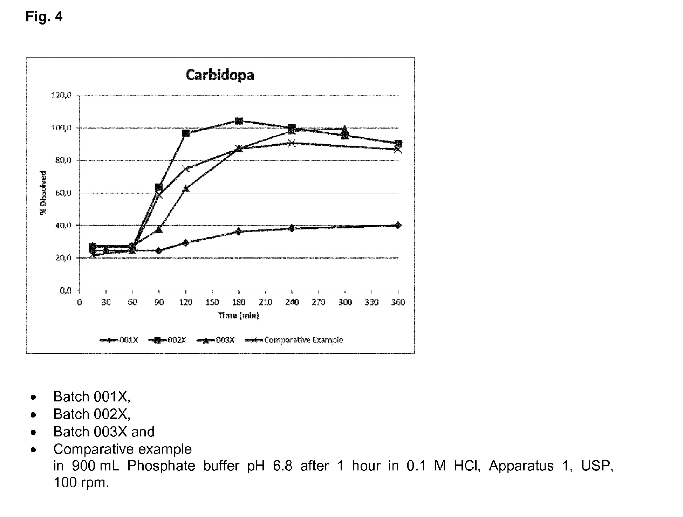

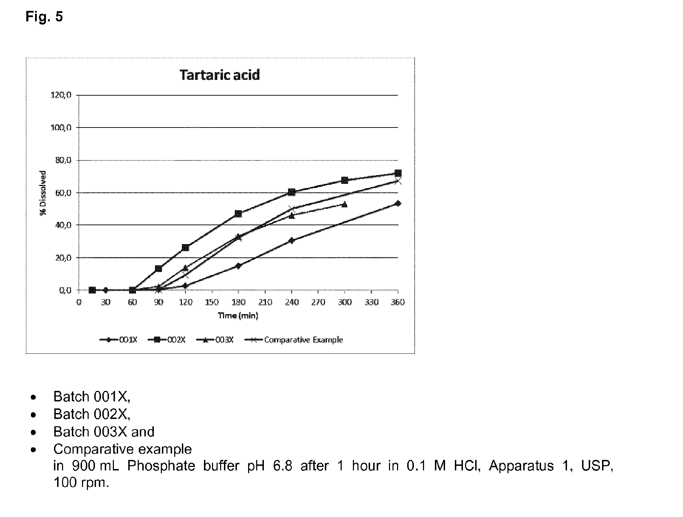

Figures 3 to 5 show the percent dissolved (mean value) of levodopa, carbidopa monohydrate, and tartaric acid.

### Evaluation of chemical stability

Chemical stability of the prepared multi-layered pellets (particles) was evaluated. Sample 002X was exposed to accelerated and long-term stability conditions for up to 6 months. With regard to degradation product formation, sample 002X is considered comparable with analogue components of the comparative example. All formed degradation products are below the justified thresholds for individual impurities.

**Stability data for sample 002X:**

| Sum of impurities | 1 month | 3 months | 6 months |
|---|---|---|---|
| 25°C/60% RH | 0.59% | 0.63% | 0.72% |
| 40°C/75% RH | 0.87% | 1.12% | 1.73% |

### Examples:

The following examples demonstrate that by applying the present invention, the dissolution profile can easily be tailored by changing the amount of each coating and/or by changing the ratio of the polymers in the respective coatings.

### Example 1: 001X

### Qualitative and quantitative composition

| Ingredients | | Strength mg/ capsule | | |
|---|---|---|---|---|
| | | 390 | 245 | |
| (**i**) | **Tartaric acid pellets** | **210.96** | **132.53** | **mg** |
| **(ii)** | **Extended release release layer (EC:HPMC 8.25:1,75.20%)** | | | |
| Ethylcellulose N10 | | 43.53 | 27.35 | mg |
| Hydroxypropylmethyl cellulose Pharmacoat 606 | | 9.21 | 5.78 | mg |
| Talc | | 5.86 | 3.68 | mg |
| #Ethanol | | 255.44 | 160.47 | mg |
| #Acetone | | 455.45 | 286.11 | mg |
| #Purified Water | | 67.66 | 42.50 | mg |
| **(iii)** | **MRS 1** | | | |
| **(iiia)** | **API layer (15% binder, 30% solids)** | | | |
| Carbidopa monohydrate | | 67.20 | 42.22 | mg |
| Levodopa | | 249.00 | 156.42 | mg |
| #Hydroxypropylmethyl cellulose Pharmacoat 606 | | 55.80 | 35.05 | mg |
| #Ethanol | | 813.75 | 511.20 | mg |
| #Purified Water | | 54.25 | 34.08 | mg |
| **(iiib)** | **extended release coating (EC:HPMC 7:3, 10%) (controlled release layer)** | | | |
| Ethylcellulose N10 | | 49.90 | 31.35 | mg |
| Hydroxypropylmethyl cellulose Pharmacoat 606 | | 21.39 | 13.43 | mg |
| Talc | | 7.92 | 4.98 | mg |
| #Ethanol | | 345.25 | 216.89 | mg |
| #Acetone | | 615.59 | 386.71 | mg |
| #Purified Water | | 91.44 | 57.44 | mg |
| **(iii)** | **MRS 2-** | | | |
| **(iiia)** | **API layer (15% binder, 30% solids)** | | | |
| Carbidopa monohydrate | | 14.98 | 9.41 | mg |
| Levodopa | | 55.50 | 34.87 | mg |
| Hydroxypropylmethyl cellulose Pharmacoat 606 | | 12.44 | 7.81 | mg |
| Ethanol | | 181.38 | 113.94 | mg |
| Purified Water | | 12.09 | 7.60 | mg |
| **(iiib)** | **extended release coating (EC:HPMC 6:4, 10%) (controlled release layer)** | | | |
| Ethylcellulose N10 | | 53.58 | 33.66 | mg |
| Hydroxypropylmethyl cellulose Pharmacoat 606 | | 35.72 | 22.44 | mg |
| Talc | | 9.92 | 6.23 | mg |
| #Ethanol | | 432.50 | 271.70 | mg |
| #Acetone | | 771.15 | 484.44 | mg |
| #Purified Water | | 114.55 | 71.96 | mg |
| **(v)** | **Delayed coating (Eudragit S: Eudragit L= 2:1, 20%)** | | | |
| Methacrylic acid copolymer, type B (Eudragit S 100) | | 105.64 | 66.36 | mg |
| Methacrylic acid copolymer, type A (Eudragit L 100) | | 52.82 | 33.18 | mg |
| Ttriethylcitrate | | 44.69 | 28.08 | mg |
| Talc | | 22.57 | 14.18 | mg |
| #Ethanol | | 1209.48 | 759.80 | mg |
| #Acetone | | 774.34 | 486.45 | mg |
| #Purified Water | | 48.57 | 30.51 | mg |
| **(vi)** | **Immediate release API layer (15% binder, 30% solids)** | | | |
| Carbidopa monohydrate | | 23.08 | 14.50 | mg |
| Levodopa | | 85.50 | 53.71 | |
| Hydroxypropylmethyl cellulose Pharmacoat 606 | | 19.16 | 12.04 | mg |
| #Ethanol | | 279.43 | 175.54 | mg |
| #Purified Water | | 18.63 | 11.70 | mg |
| Hard gelatin capsule | | 1 | 1 | pc |
| | Total | **1256.37** | 789.26 | mg |

Carbidopa is in the form of carbidopa monohydrate, the amount in each API layer corresponds to 62.25, 13.8 and 21.34 mg of carbidopa in API layer 1, 2 and 3, respectively.

### #: Removed during drying

### Process:

The whole manufacture is carried out in fluid bed dryer Huettlin Kögel Coater. Several coating dispersions were prepared and separately sprayed onto tartaric acid pellets cores.

Preparation of extended release coating dispersion: ethylcellulose and hypromellose at appropriate weight ratio were dissolved in mixture of ethanol, acetone and water. Afterwards, talc was added into polymer dispersion and mixed thoroughly throughout the coating process. The solids represented 7 w/w% of extended release coating dispersion.

Preparation of API coating dispersion: hypromellose was dissolved in mixture of ethanol and water. Afterwards, LD and CD at a weight ratio 4:1 were suspended in hypromellose solution and mixed thoroughly throughout the coating process. The solids represented 30 w/w% of API coating dispersion.

Preparation of delayed coating dispersion: Eudragit S and L at appropriate weight ratio were dissolved in a mixture of ethanol, acetone and water. Separately, triethylcitrate (TEC) and talc were dispersed in the same mixture of solvents. Both dispersions were combined and mixed thoroughly throughout the coating process. The solids represented 10 w/w% of extended release coating dispersion.

Coating dispersions were sprayed onto tartaric acid pellets in fluid bed dryer in the following order:
1. 1.20 w/w % of extended release coating at ethylcellulose and hypromellose weight ratio 8.25:1.75
2. MRS 1:
   a) API layer representing approximately 64% of total API dose
   b) 10 w/w % of extended release coating at ethylcellulose and hypromellose weight ratio 7:3
3. MRS 2:
   a) API layer representing approximately 14% of total API dose
   b) 10 w/w % of extended release coating at ethylcellulose and hypromellose weight ratio 6:4
4. 10 w/w % of delayed coating at Eudragit S:L weight ratio 2:1
5. Immediate release API layer representing approximately 22% of total API dose

After each coating the pellets were dried to remove residual solvents.

### Example 2: 002X

### Qualitative and quantitative composition

| Ingredients | | Strength mg/ capsule | | |
|---|---|---|---|---|
| | | 390 mg | 245 mg | |
| (**i**) | **Tartaric acid pellets** | 210.96 | 132.53 | mg |
| **(ii)** | **Extended release layer (EC:HPMC 8.25:1.75, 20%)** | | | |
| Ethylcellulose N10 | | 43.53 | 27.35 | mg |
| Hydroxypropylmethyl cellulose Pharmacoat 606 | | 9.21 | 5.78 | mg |
| Talc | | 5.86 | 3.68 | mg |
| #Ethanol | | 255.44 | 160.47 | mg |
| #Acetone | | 455.45 | 286.11 | mg |
| #Purified Water | | 67.66 | 42.50 | mg |
| **(iii)** | **MRS 1** | | | |
| **(iiia)** | **API layer (15% binder, 30% solids)** | | | |
| Carbidopa monohydrate | | 67.01 | 42.09 | mg |
| Levodopa | | 249.00 | 156.42 | mg |
| #Hydroxypropylmethyl cellulose Pharmacoat 606 | | 51.01 | 32.04 | mg |
| #Ethanol | | 802.85 | 504.35 | mg |
| #Purified Water | | 53.52 | 33.62 | mg |
| (iiib) | **Extended release coating (EC:HPMC 5.5:4.5, 7.5%) (controlled release layer)** | | | |
| Ethylcellulose N10 | | 28.62 | 17.98 | mg |
| Hydroxypropylmethyl cellulose Pharmacoat 606 | | 23.42 | 14.71 | mg |
| Talc | | 5.78 | 3.63 | mg |
| #Ethanol | | 252.04 | 158.34 | mg |
| #Acetone | | 449.39 | 282.31 | mg |
| #Purified Water | | 66.76 | 41.94 | mg |
| **(iii)** | **MRS 2-API layer** | | | |
| **(iiia)** | **API layer (15% binder, 30% solids)** | | | |
| Carbidopa monohydrate | | 14.94 | 9.38 | mg |
| Levodopa | | 55.50 | 34.87 | mg |
| Hydroxypropylmethyl cellulose Pharmacoat 606 | | 11.37 | 7.14 | mg |
| Ethanol | | 178.95 | 112.42 | mg |
| Purified Water | | 11.93 | 7.49 | mg |
| **(iiib)** | **Extended release coating (EC:HPMC 3:7, 7.5%) (controlled release layer)** | | | |
| Ethylcellulose N10 | | 19.01 | 11.94 | mg |
| Hydroxypropylmethyl cellulose Pharmacoat 606 | | 44.35 | 27.86 | mg |
| Talc | | 7.04 | 4.42 | mg |
| #Ethanol | | 306.88 | 192.78 | mg |
| #Acetone | | 547.16 | 343.73 | mg |
| #Purified Water | | 81.28 | 51.06 | mg |
| **(v)** | **Delayed coating (Eudragit S: Eudragit L= 2:1, 10%)** | | | |
| Methacrylic acid copolymer, type B (Eudragit S 100) | | 44.30 | 27.83 | mg |
| Methacrylic acid copolymer, type A (Eudragit L 100) | | 22.15 | 13.91 | mg |
| Ttriethylcitrate | | 18.74 | 11.77 | mg |
| Talc | | 9.47 | 5.95 | mg |
| #Ethanol | | 506.94 | 318.46 | mg |
| #Acetone | | 324.56 | 203.89 | mg |
| #Purified Water | | 20.36 | 12.79 | mg |
| **(vi)** | **Immediate release API layer (15% binder, 30% solids)** | | | |
| Carbidopa monohydrate | | 23.01 | 14.45 | mg |
| Levodopa | | 85.50 | 53.71 | |
| Hydroxypropylmethyl cellulose Pharmacoat 606 | | 17.52 | 11.00 | mg |
| #Ethanol | | 275.68 | 173.18 | mg |
| #Purified Water | | 18.38 | 11.55 | mg |
| Hard gelatin capsule | | 1 | | pc |
| | Total | **1072.52** | 673.76 | mg |

Carbidopa is in the form of carbidopa monohydrate, the amount in each API layer corresponds to 62.25, 13.8 and 21.34 mg of carbidopa in API layer 1, 2 and 3, respectively.

### #: Removed during drying

Pellets presented in example 2 were prepares using the same procedure as described in Example 1, except the ratio of polymers in extended release and delayed release coating as well as amount of these coatings were changed.

### Example 3: 003X

### Qualitative and quantitative composition

| Ingredients | | Strength mg/ capsule | | |
|---|---|---|---|---|
| | | 390 | 245 | |
| **(i)** | **Tartaric acid pellets** | **210.96** | **132.53** | **mg** |
| **(ii)** | **Extended release release layer (EC:HPMC 8.25:1.75, 20%)** | | | |
| Ethylcellulose N10 | | 43.53 | 27.35 | mg |
| Hydroxypropylmethyl cellulose Pharmacoat 606 | | 9.21 | 5.78 | mg |
| Talc | | 5.86 | 3.68 | mg |
| #Ethanol | | 255.44 | 160.47 | mg |
| #Acetone | | 455.45 | 286.11 | mg |
| #Purified Water | | 67.66 | 42.50 | mg |
| **(iii)** | **MRS 1** | | | |
| **(iiia)** | **API layer (15% binder, 30% solids)** | | | |
| Carbidopa monohydrate | | 67.01 | 42.09 | mg |
| Levodopa | | 249.00 | 156.42 | mg |
| #Hydroxypropylmethyl cellulose Pharmacoat 606 | | 51.01 | 32.04 | mg |
| #Ethanol | | 802.85 | 504.35 | mg |
| #Purified Water | | 53.52 | 33.62 | mg |
| **(iiib)** | **Extended release coating (EC:HPMC 5.5:4.5, 12%) (controlled release)** | | | |
| Ethylcellulose N10 | | 48.14 | 30.24 | mg |
| Hydroxypropylmethyl cellulose Pharmacoat 606 | | 39.39 | 24.74 | mg |
| Talc | | 9.73 | 6.11 | mg |
| #Ethanol | | 423.90 | 266.30 | mg |
| #Acetone | | 755.82 | 474.81 | mg |
| #Purified Water | | 112.27 | 70.53 | mg |
| **(iii)** | **MRS 2-** | | | |
| **(iiia)** | **API layer (15% binder, 30% solids)** | | | |
| Carbidopa monohydrate | | 14.94 | 9.38 | mg |
| Levodopa | | 55.50 | 34.87 | mg |
| Hydroxypropylmethyl cellulose Pharmacoat 606 | | 11.37 | 7.14 | mg |
| Ethanol | | 178.95 | 112.42 | mg |
| Purified Water | | 11.93 | 7.49 | mg |
| **(iiib)** | **Extended release coating (EC:HPMC 3:7, 12%) (controlled release layer)** | | | |
| Ethylcellulose N10 | | 33.58 | 21.10 | mg |
| Hydroxypropylmethyl cellulose Pharmacoat 606 | | 78.36 | 49.22 | mg |
| Talc | | 12.44 | 7.81 | mg |
| #Ethanol | | 542.15 | 340.58 | mg |
| #Acetone | | 966.65 | 607.26 | mg |
| #Purified Water | | 143.59 | 90.21 | mg |
| **(v)** | **Delayed coating (Eudragit S: Eudragit L= 2:1, 15%)** | | | |
| Methacrylic acid copolymer, type B (Eudragit S 100) | | 78.07 | 49.04 | mg |
| Methacrylic acid copolymer, type A (Eudragit L 100) | | 39.03 | 24.52 | mg |
| Ttriethylcitrate | | 33.03 | 20.75 | mg |
| Talc | | 16.68 | 10.48 | mg |
| #Ethanol | | 893.41 | 561.24 | mg |
| #Acetone | | 571.99 | 359.32 | mg |
| #Purified Water | | 35.88 | 22.54 | mg |
| **(vi)** | **Immediate release API layer (15% binder, 30% solids)** | | | |
| Carbidopa monohydrate | | 23.01 | 14.45 | mg |
| Levodopa | | 85.50 | 53.71 | |
| Hydroxypropylmethyl cellulose Pharmacoat 606 | | 17.52 | 11.00 | mg |
| #Ethanol | | 275.677 | 173.18 | mg |
| #Purified Water | | 18.38 | 11.55 | mg |
| Hard gelatin capsule | | 1 | 1 | pc |
| | Total | **1238.07** | 777.76 | mg |

Carbidopa is in the form of carbidopa monohydrate, the amount in each API layer corresponds to 62.25, 13.8 and 21.34 mg of carbidopa in API layer 1, 2 and 3, respectively. #: Removed during drying

### Process:

Pellets presented in example 3 were prepared using the same procedure as described in Example 1, except the ratio of polymers in the extended release and the delayed release coating as well as amount of these coatings were changed.

### Comparative Example

Comparative example A was prepared based on composition and procedure disclosed in US8377474 B2 - Example 9. The only difference is substitution of isopropyl alcohol with ethanol, higher amount of PVP in ultrafast component, use of 0.9 mm instead of 1.0 mm extrusion screen and upper mesh screen size for screening of the pellet cores.

| | IPX066 AH 6(A) 245 mg mg/capsule |
|---|---|
| **Component I** | |
| Carbidopa USP | 14.60* |
| Levodopa USP | 53.71 |
| Croscarmellose Sodium | 5.31 |
| Povidone | 3,14 |
| Magnesium Stearate | 0.38 |
| Purified Water, USP | N/A** |

| **Component II** | |
|---|---|
| Carbidopa USP | 9.42* |
| Levodopa USP | 34.87 |
| Microcrystalline Cellulose NF | 6.43 |
| Mannitol NF | 6.43 |
| Sodium Starch Glycolate NF | 3.21 |
| Sodium Lauryl Sulfate NF | 3.21 |
| Povidone USP | 0.63 |
| Methacrylic acid copolymer, Type A NF (Eudragit L 100) | 0.79 |
| Methacrylic acid Copolymer, Type B NF (Eudragit S 100) | 1.57 |
| Triethyl Citrate NF | 0.67 |
| Talc USP | 0.68 |
| Acetone, NF | N/A** |
| Ethanol, USP | N/A** |
| Purified Water, USP | N/A** |

| **Component III** | |
|---|---|
| Carbidopa USP | 42.22* |
| Levodopa USP | 156.42 |
| Microcrystalline Cellulose NF | 85.13 |
| Methacrylic acid copolymer, Type A NF (Eudragit L 100) | 3.42 |
| Methacrylic acid Copolymer, Type B NF (Eudragit S 100) | 7.02 |
| Triethyl Citrate NF | 3.01 |
| Talc USP | 2.99 |
| Acetone, NF | N/A** |
| Ethanol, USP | N/A** |
| Purified Water, USP | N/A** |

| **Component IV** | |
|---|---|
| Tartaric acid NF | 132.53 |
| Microcrystalline Cellulose NF | 33.07 |
| Ethylcellulose NF | 15.27 |
| Hypromellose Type 2910 USP | 3.23 |
| Methacrylic acid copolymer, Type A NF (Eudragit L 100) | 10.63 |
| Methacrylic acid Copolymer, Type B NF (Eudragit S100) | 21.52 |
| Triethyl Citrate NF | 9.25 |
| Talc USP | 5.78 |
| Acetone, NF | N/A** |
| Ethanol, USP | N/A** |
| Purified Water, USP | N/A** |
| Hard Gelatin Capsule | I unit |
| **Total Capsule Fill weight** | 675.19 |

### CD/LD Component I

The required amounts of carbidopa, levodopa, croscarmellose sodium, and povidone were charged into a high shear granulator and mixed. The mixed powder was granulated by adding the purified water. The granules were discharged and dried. The granules were sieved through 0.7 mm mesh using oscillating sieve. The collected granules were blended with magnesium stearate.

### CD/LD Component II:

Carbidopa, levodopa, and microcrystalline cellulose, mannitol, Sodium Starch Glycolate, Sodium Lauryl Sulfate, and Povidone were charged in a high shear granulator and granulated with purified water. The granulated wet mass was extruded in an extruder with 0.9 mm size screen. The extrudate was charged and spheronized in a spheronizer. The spheres were dried and screened with 16 and 25 mesh screens. The enteric coating solution was prepared by dissolving Eudragit S100 and Eudragit L100 at the weight ratio of 2:1 and triethyl citrate in ethanol and acetone solution. Talc was then dispersed into the polymer solution and mixed continuously throughout the coating process. The CD/LD pellets were spray coated using the coating dispersion in a coater. The coated pellets were dried and then screened through a 14 mesh screen. The screened CD/LD pellets were blended with talc.

### CD/LD component III:

CD, LD, and microcrystalline cellulose (Avicel PH-101) were charged into a high shear granulator and granulated with purified water. The granulated wet mass was extruded in an extruder with 0.9 mm hole size screen. The extrudate was charged and spheronized in a spheronizer. The spheres were dried and screened with 16 and 25 mesh screens.

Eudragit S100 and Eudragit L100 (at the weight ratio of 2:1) and triethyl citrate were dissolved in ethanol and acetone solution. The mixture was mixed until dissolved. Talc was dispersed into the polymer solution and mixed continuously throughout the coating process. The CD/LD pellets were spray-coated. The coated pellets were dried. The dried, coated CD/LD pellets were screened through a 14 mesh screen. The screened CD/LD pellets were charged and blended with talc.

### Component IV

Tartaric acid and microcrystalline cellulose was charged in a high shear mixer and granulated with purified water. The wet mass was extruded in an extruder with 0.9 mm hole size screen. The extrudate was charged and spheronized in a spheronizer and the resulting pellets dried. The dried pellets were passed through 16 and 25 mesh screens.

A seal coat solution was prepared by dissolving hypromellose (Pharmacoat 606) and ethylcellulose in alcoholic solution. The seal coat solution was applied to the tartaric acid pellets in a coater. The pellets were dried and the dried pellets passed through a 14 mesh screen. An enteric coating solution was prepared by dissolving Eudragit S100 and Eudragit L100 at the weight ratio of 2:1 and triethyl citrate in ethanol and acetone solution. Talc was dispersed into the polymer solution and mixed continuously throughout the coating process. The screened tartaric acid pellets were charged in a coater and spray coated with the enteric coating dispersion. The coated pellets were dried and screened through a 14 mesh screen. The TA pellets were charged and blended with talc.

The required amounts of pellets were filled into gelatin capsules according to specific fill weight.

## Claims

1. A multilayered coated particle comprising, from the inside to the outside, in the order from i) to vi):
i) a core comprising one or more carboxylic acids;
ii) a controlled release coating layer
comprising one or more rate-controlling polymer(s) which is/are practically insoluble at a pH in the range of from 1 to 8, and optionally one or more coating additives;
iii) one or more Modified Release System (MRS) layer combination(s), wherein each MRS layer combination comprises
(iiia) an API coating layer,
comprising levodopa and carbidopa and one or more immediate release or modified release film forming polymers and/or one or more coating additives, or
an API coating layer, which is a combination of two API coating layers,
one comprising levodopa and one comprising carbidopa, and each comprising one or more immediate release or modified release film forming polymers and/or one or more coating additives;
and
(iiib) a controlled release coating layer,
comprising one or more rate-controlling polymer(s) which is/are practically insoluble at a pH in the range of from 1 to 8 and optionally one or more coating additives;
wherein, from the inside to the outside, coating layer (iiia) precedes coating layer (iiib);
(iiic) optionally further a coating layer combination having two layers (α) and
(β), wherein layer (α) comprises one or more carboxylic acid(s), one or more film forming polymers and optionally anti-tacking agent and plasticizer, and layer (β) is a separating seal coat comprising one or more immediate release or modified release film forming polymers and optionally one or more coating additives,
wherein said layer α of the optional coating layer combination (iiic) can be present either prior or after the API coating layer (iiia),
with the proviso that in case optional layer combination (iiic) is provided, the separating seal coat (β) is positioned between layer (α) and the API coating layer (iiia);
wherein the MRS layer combination(s) (iii) can be present up to 5 times in a consecutive manner;
iv) optionally, a further API coating layer as defined in (iiia);
v) a controlled release coating layer (v) comprising one or more rate controlling polymer(s) with pH dependent solubility or a mixture of rate controlling polymer(s) with pH dependent solubility and rate controlling polymer(s) which is/are practically insoluble at a pH in the range of from 1 to 8, and one or more coating additives, preferably directly positioned on the optional further API coating layer (iv), or on the Modified Release System (MRS) layer combination(s) (iii), if API coating layer (iv) is absent; and
vi) an immediate release API coating layer comprising levodopa, carbidopa monohydrate, one or more immediate release film forming polymers and/or one or more coating additives, preferably not covered by a further layer.

2. The multilayered coated particle according to claim 1, wherein the carboxylic acid is tartaric acid, and/or wherein the coating layers (ii), (iiib), and (v) additionally comprise talc, and/or wherein the polymers and coating additives, present in the same layer, are different from each other.

3. The multilayered coated particle according to claim 1 or 2, wherein
(I) at least one, preferably all, of said rate-controlling polymer(s) which is/are practically insoluble at a pH in the range of from 1 to 8 is/are selected from the group consisting of cellulose derivatives such as ethylcellulose; polymethacrylate esters/co-polymers, such as meth- /acrylate copolymers with trimethyl-ammonioethyl-methacrylate as functional group, preferably polymers from the Eudragit® RL series, such as Eudragit® RL 30 D, PO, 100, 12,5, and polymers from the Eudragit® RS series, such as Eudragit® RS 30 D, PO, 100, 12,5), and such as neutral polymers of meth- /acrylates, preferably polymers from the Eudragit® NE series, such as Eudragit® NE 30 D, and 40 D, and polymers from the Eudragit® NM series, such as Eudragit® NM 30 D; waxes; lipids; polyvinyl chloride; polyethylene; vinyl acetate/vinyl chloride copolymers; and polyamides;
(II) the rate-controlling polymer(s) with pH-dependent solubility is/are pharmaceutically acceptable and have a pH dependent solubility within the pH range of from 1 to 8; preferably, at least one, preferably all, of said rate-controlling polymer(s) is/are selected from the group consisting of cellulose acetate phthalate (CAP); cellulose acetate trimellitate (CAT); carboxymethyl ethylcellulose (CMEC); hydroxypropylmethylcellulose phthalate (HPMCP); methacrylic acid and methyl methycrylate copolymers; fatty acids; waxes; shellack; and polyvinyl acetate phthalate (PVAP);
(III) the coating additive(s) is/are pharmaceutically acceptable and at least one, preferably all, is/are selected from the group consisting of plasticizers such as triethylcitrate (TEC); appearance modifiers such as pigments; anti-tacking agents; permeability-modifying agents, e.g. soluble polymers such as polyvinylpyrrolidone (PVP), low viscosity HPMC, preferably Pharmacoat 606, low viscosity hydroxypropylcellulose (HPC), high viscosity HPMC, high viscosity HPC, or polysaccharides;
(IV) the film forming polymer(s) is/are pharmaceutically acceptable and is/are able to form a film and bind solids in dispersions into a coating, preferably the film forming polymer is an immediate release film forming polymer that has essentially no delaying or sustaining influence on API release, such as low viscosity HPMC, preferably Pharmacoat 606, low viscosity HPC, or PVP; or the film forming polymer(s) is/are a modified release film forming polymer that is able to alter the API release, such as polymers that are practically insoluble in water, such as cellulose derivatives, polymethacrylates, copolymers of methacrylic acid; polymers that form a matrix or gel, such as polysaccharides; or hydrophilic polymers that swell in contact with water and thereby form a hydrophilic matrix or a gel, such as high viscosity HPMC, preferably Methocel K4M - K100M, high viscosity HPC, preferably Klucel MXF.

4. The multilayered coated particle according to any of claims 1 to 3, wherein
(A) the controlled release coating layer (ii) comprises ethylcellulose, preferably ethylcellulose N10, as a rate-controlling polymer and low viscosity HPMC, preferably HPMC Pharmacoat 606, as a coating additive;
(B) the coating layer (iiia) comprises low viscosity HPMC, preferably HPMC Pharmacoat 606, as film forming polymer;
(C) the coating layer (iiib) comprises ethylcellulose, preferably ethylcellulose N10, as a rate-controlling polymer and low viscosity HPMC, preferably HPMC Pharmacoat 606, as coating additive;
(D) the coating layer (iiic), if present, comprises low viscosity HPMC, preferably HPMC Pharmacoat 606, or PVP as a film-forming polymer in the layer comprising carboxylic acid(s), and anti-tacking agent and/or plasticizer in the seal coat;
(E) the coating layer (v) comprises two different types of methacrylic acid copolymers as rate controlling polymers, preferably methacrylic acid copolymer Type B (e.g. Eudragit® S 100) and methacrylic acid copolymer Type A (e.g. Eudragit® L 100), and TEC and talc as coating additive; and/or
(F) the coating layer (vi) comprises HPMC Pharmacoat 606 as film forming polymer, and/or one or more disintegrants as coating additive(s), such as crospovidone, croscarmellose sodium, or sodium starch glycolate.

5. The multilayered coated particle according to claim 4, wherein the weight ratio of low viscosity HPMC, preferably HPMC Pharmacoat 606, to ethylcellulose, preferably ethylcellulose N10,
(e) in coating layer (ii) is in a range of from 1:0.5 to 1:15;
(ee) in coating layer (iiib) is in a range of from 1:0.5 to 1:15.

6. The multilayered coated particle according to any of claims 1 to 5, wherein the weight ratio of the two different types of methacrylic acid copolymers as rate controlling polymers, preferably Eudragit S 100 and Eudragit L 100, is in a range of from 1:0.05 to 0.05:1, and/or wherein the MRS coating layer combination(s) comprise(s) carbidopa monohydrate and levodopa in an amount of equal to or more than 60 wt.-% and equal to or less than 90 wt.-%, based on the total amount of carbidopa monohydrate and levodopa being present in the multilayered coated particle.

7. The multilayered coated particle according to any of claims 1 to 6, wherein the carbidopa monohydrate and the levodopa respectively have a maximum mean particle size of d(0.9) of about 35µm, preferably the carbidopa monohydrate and the levodopa respectively have a mean particle size of d(0.9) in the range of from 1 µm to 30 µm, as determined by laser diffraction technology on the basis of the volumetric mean value.

8. Pharmaceutical composition comprising multilayered coated particles according to any of claims 1 to 7, and at least one further pharmaceutically acceptable excipient selected from the group consisting of filler, binder, lubricant, glidant, and disintegrant.

9. An oral dosage form, preferably a tablet, sachet or a capsule, more preferably a capsule, comprising the pharmaceutical composition according to claim 8 or one or more of the multilayered coated particles according to any of claims 1 to 7.

10. Process for the preparation of a multilayered coated particle as defined in any of claims 1-7, comprising the steps of
a) providing the core (i);
b) applying the controlled release coating layer (ii) by using a dispersion comprising one or more rate-controlling polymers and optionally one or more coating additives;
c) applying the API coating layer (iiia) by using an organic or aqueous dispersion or a dispersion containing organic solvent(s) and water, comprising levodopa and carbidopa monohydrate, and one or more immediate release or modified release film forming polymers and/or one or more coating additives;
d) applying the controlled release coating layer (iiib) by using a coating dispersion comprising one or more rate-controlling polymer(s) which is/are practically insoluble at a pH in the range of from 1 to 8, and optionally one or more coating additives;
e) optionally, applying layer (α) of the coating layer combination (iiic) by using a dispersion of carboxylic acid and one or more film forming polymers, and optionally anti-tacking agent and plasticizer; and
applying layer (β) of the coating layer combination (iiic) directly prior or after the API coating layer (iiia) by using a coating dispersion comprising one or more immediate release or modified release film forming polymers and/or one or more coating additives;
wherein steps c) to e) can be repeated up to 4 times;
f) optionally, applying the API coating layer (iv) by using a dispersion comprising levodopa and carbidopa monohydrate, and one or more immediate release or modified release film forming polymers and/or one or more coating additives;
g) applying the controlled release coating layer (v) by using a dispersion comprising one or more rate controlling polymers with pH dependent solubility or a mixture of polymers with pH dependent solubility and polymer(s) which is/are practically insoluble at a pH in the range of from 1 to 8, and one or more coating additives; and
h) applying the immediate release API coating layer (vi) by using a dispersion comprising carbidopa monohydrate, levodopa, one or more immediate release film forming polymers, and/or one or more coating additives.

11. The process of claim 10, wherein the carboxylic acid is tartaric acid, or wherein the core either comprises, or consists of, tartaric acid crystals, and/or is prepared by layering or extrusion spheronisation.

12. The process according to claim 10 or 11, wherein the respective layers are applied by spray coating technique, and/or wherein the carbidopa monohydrate and the levodopa respectively have a maximum mean particle size of d0.9 of about 35µm, preferably the carbidopa monohydrate and the levodopa respectively have a mean particle size of d0.9 in the range of from 1 µm to 30 µm, as determined by laser diffraction technology on the basis of the volumetric mean value.

13. The process according to any of claims 10-12 for preparing a multilayered coated particle according to claim 4, wherein the dispersion used for preparing the controlled release coating layer (ii) has a solid content in a range of from 3 to 15 w/w% and an ethyl cellulose to HPMC ratio in a range of from 1:1 to 10:1 and/or wherein the ratio of the weight of the controlled release coating layer (ii) to the weight of the pellet core (i) plus the weight of the controlled release coating layer (ii) is in a range of from 5% to 40%, preferably 10% to 30%, more preferably is in a range of from 15 to 25%, further preferred is about 20%.

14. The process according to any of claims 10-13, wherein the dispersion used for preparing the/one of the MRS layer combination(s) (iii) has a solid content in a range of from 3 to 15 w/w% and an ethyl cellulose to HPMC ratio in a range of from 2:1 to 10:1 and/or wherein the ratio of the weight of the MRS layer combination(s) (iii) to the weight of the pellet core (i) plus the weight of the controlled release coating layer (ii) plus the weight of the MRS layer combination(s) (iii) is in a range of from 5% to 40%, preferably 5% to 20%, more preferably is in a range of from 7 to 15%, further preferred is about 10%.

15. The multilayered coated particle according to any of claims 1 to 7, the pharmaceutical composition according to claim 8, or the oral dosage form of claim 9, for use in a method of treating Parkinson's disease.
